# FASCICULE DE BREVET EUROPEEN

(11) **EP 0 678 088 B1**
(45) Date de publication et mention de la délivrance du brevet: **09.10.1996**
(21) Numéro de dépôt: 94903939.0
(22) Date de dépôt: 03.01.1994
(51) Int. Cl.: C07D 207/16, C07K 5/062, A61K 31/40, A61K 38/05, C07D 207/08, C07D 207/09

(54) **N-ACYL PYRROLIDINES ET DES MEDICAMENTS POUR LE TRAITEMENT OU LA PREVENTION DES DESORDRES LIES A LA CKK ET LA GASTRINE**
N-ACYLPYRROLIDINE UND ARZNEIMITTEL ZUR BEHANDLUNG ODER PROPHYLAXE VON MIT CKK UND GASTRIN IN ZUSAMMENHANG STEHENDEN KRANKHEITEN
N-ACYL PYRROLIDINES AND DRUGS FOR THE TREATMENT OR PREVENTION OF CHOLECYSTOKININ AND GASTRIN-RELATED DISORDERS

(30) Priorité: 07.01.1993 FR 9300077
(43) Date de publication de la demande: 25.10.1995
(73) Titulaire: RHONE-POULENC RORER S.A., 92160 Antony (FR)
(72) Inventeur: CAPET, Marc, F-94320 Thiais (FR); DUBROEUCQ, Marie-Christine, F-95880 Enghien-les-Bains (FR)
(74) Mandataire: Savina, Jacques
(86) Numéro de dépôt international: FR9400008
(87) Numéro de publication internationale: WO9415914

(56) Documents cités:
- EP-A- 0 124 317
- WO-A-91/13907
- FR-A- 2 678 938
- TETRAHEDRON LETTERS. vol. 34, no. 3 , 1993 , OXFORD GB pages 381 - 384 Magaard, Victoria W.; Sanchez, Robert M.; Bean, John W.; Moore, Michael L. 'A convenient synthesis of the conformationally constrained amino acid 5,5-dimethylproline'
- TETRAHEDRON LETTERS. vol. 34, no. 10 , 1993 , OXFORD GB pages 1665 - 1668 Baldwin, Jack E.; Hulme, Christopher; Edwards, Alison J.; Schofield, Christopher J.; Parkes, Kevin E. B. 'Synthesis of a bicyclic .gamma.-lactam dipeptide analog'

## Description

La présente invention concerne des dérivés de formule : 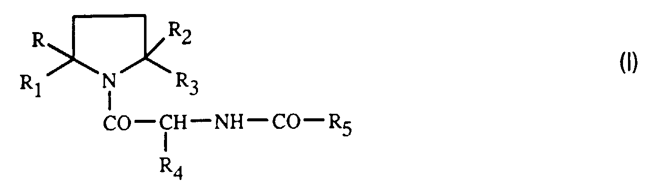 leurs sels, leur préparation et les médicaments les contenant.

Dans la formule (I),
R représente un atome d'hydrogène ou un radical alkyle, cycloalkyle, phénylalkyle ou phényle éventuellement substitué par un ou plusieurs substituants choisis parmi les atomes d'halogène et les radicaux alkyle et alcoxy,
R₁ représente un radical alkyle contenant 1 à 12 atomes de carbone, en chaîne droite ou ramifiée et éventuellement mono ou polyinsaturé, cycloalkyle contenant 3 à 12 atomes de carbone et éventuellement mono ou polyinsaturé, polycycloalkyle contenant 6 à 12 atomes de carbone et éventuellement mono ou polyinsaturé, phénylalkyle dont le noyau phényle est éventuellement substitué (par un ou plusieurs substituants choisis parmi les radicaux alkyle, alcoxy ou les atomes d'halogène), diphénylalkyle, cinnamyle, pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₇R_{8,} -NH-CO-CH₃, trifluorométhyle ou trifluorométhoxy,
R₂ représente une chaîne -(CH₂)ₙ-CO-R₆, -(CH₂)ₘ-O-CO-R"₆, -(CH₂)ₘ-NR₉R₁₀ ou un radical oxazolinyle éventuellement substitué par un ou plusieurs radicaux alkyle ou alkyl-3 oxadiazolyle,
R₃ représente un atome d'hydrogène ou un radical alkyle, cycloalkyle ou phénylalkyle éventuellement substitué par un ou plusieurs substituants choisis parmi les atomes d'halogène et les radicaux alkyle et alcoxy,
R₄ représente un atome d'hydrogène ou un radical alkyle,
R₅ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₁, -SO₂-NH-SO₂-R₁₁, -CO-NH-CO-R₁₁ -CO-NH-SO₂-R₁₁, -B(OH)₂, -C(NH₂)=NOH, -SO₂-NH-R₁₂, -CO-NH-R₁₂, 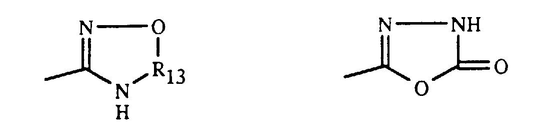 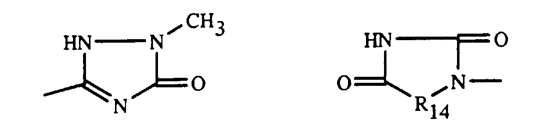 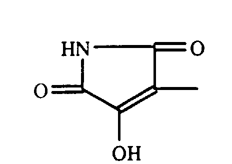 ou diméthyl-2,2 dioxo-4,6 dioxanne-1,3-yl-5,
R₆ représente un radical hydroxy, alcoxy, cycloalkyloxy, cycloalkylalkyloxy, phényle ou -NR₉R₁₀,
R"₆ représente un radical alcoxy, cycloalkyloxy, cycloalkylalkyloxy, phényle ou -NR₉R₁₀,
R₇ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
R₈ représente un radical alkyle, phénylalkyle ou phényle éventuellement substitué par un ou plusieurs substituants choisis parmi les atomes d'halogène et les radicaux alkyle, alcoxy et alkylthio,
ou bien R₇ et R₈ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,
R₉ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
R₁₀ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
ou bien R₉ et R₁₀ forment ensemble avec l'atome d'azote auquel ils sont rattachés un hétérocycle mono ou polycyclique saturé ou insaturé contenant 4 à 9 atomes de carbone et un ou plusieurs hétéroatomes (O, N, S) et éventuellement substitué par un ou plusieurs radicaux alkyle,
R₁₁ représente un radical alkyle, cycloalkyle, trifluorométhyle, phényle éventuellement substitué par un ou plusieurs substituants choisis parmi les radicaux cyano, alcoxy, nitro, amino et les atomes d'halogène,
R₁₂ représente un radical tétrazolyl-5,
R₁₃ représente C=O ou S=O,
R₁₄ représente O ou C=O,
n est égal à 0, 1 ou 2,
m est égal à ou 2,
X représente un atome d'hydrogène, un radical alkyle ou phénylalkyle,
alk représente un radical alkyle ou alkylène,
alk' représente un radical hydroxyalkyle, hydroxyalkylène, alkoxyalkyle ou alkoxyalkylène
étant entendu que n est différent de 0 lorsque R et R₃ représentent chacun un atome d'hydrogène et R₁ représente un radical pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₇R_{8,} -NH-CO-CH₃, trifluorométhyle ou trifluorométhoxy; cette dernière mesure étant nécessaire pour exclure les composés déjà divulgués dans l'art antérieur FR-A-2 678 938 (WO-A-93/01167, EP-A-527 069).

Dans les définitions qui précèdent et celles qui seront citées ci-après, sauf mention contraire, les radicaux alkyle, alkylène et alcoxy et les portions alkyle, alkylène et alcoxy contiennent 1 à 4 atomes de carbone en chaîne droite ou ramifiée, les radicaux ou portions acyle contiennent 2 à 4 atomes de carbone et les radicaux et portions cycloalkyle contiennent 3 à 6 atomes de carbone.

Lorsque R₁ représente un radical alkyle insaturé, celui-ci est de préférence un radical isopropényle.

Lorsque R₁ représente un radical cycloalkyle, celui-ci est de préférence un radical cyclohexyle.

Lorsque R₁ représente un radical cycloalkyle insaturé, celui-ci est de préférence un radical tétrahydrophényle, cyclopentadiènyle ou dihydrophényle.

Lorsque R₁ représente un radical polycycloalkyle, celui-ci est de préférence un radical norbornyle ou adamantyle.

Lorsque R₁ représente un radical polycycloalkyle insaturé, celui-ci est de préférence un radical norbornènyle.

Lorsque R₇ et R₈ forment avec l'atome d'azote auquel ils sont rattachés un hétérocycle, celui-ci est de préférence un cycle pipéridino éventuellement substitué par un ou plusieurs radicaux alkyle ou un cycle tétrahydro-1,2,3,4 quinoléine.

Lorsque R₉ et R₁₀ forment avec l'atome d'azote auquel ils sont rattachés un hétérocycle, celui-ci est de préférence un cycle pipéridino, perhydroazépinyl-1, tétrahydro-1,2,3,6 pyridyl-1, tétrahydro-1,2,3,4 quinolyl-1, pyrrolidinyl-1, tétrahydro-1,2,3,4 isoquinolyl-2, thiomorpholino ou indolinyl-1, ces cycles pouvant être éventuellement substitués par au moins un radical alkyle.

Les composés de formule (I) comportant un ou plusieurs centres asymétriques présentent des formes isomères. Ces isomères font également partie de l'invention.

Les composés de formule (I) pour lesquels R₅ représente un radical phénylamino éventuellement substitué peuvent être préparés par action d'un dérivé réactif de l'acide carbamique, obtenu éventuellement in situ par action d'un dérivé réactif de l'acide carbonique choisi parmi le N,N'-diimidazole carbonyle, le phosgène, le diphosgène, le triphosgène et le chloroformiate de p-nitrophényle sur un dérivé de formule : 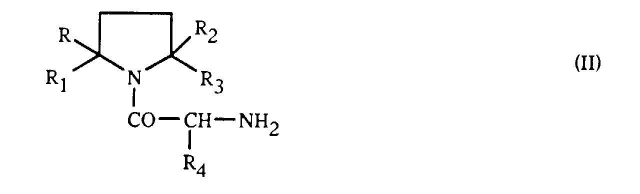 dans laquelle R, R₁, R₂, R₃ et R₄ ont les mêmes significations que dans la formule (I), sur une aniline dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H (sous forme de sel), -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-N H-CO-R₁₁, SO₂-NH-SO₂-R₁₁, -CO-NH-CO-R₁₁, -CO-NH-SO₂-R₁₁, -B(OH)₂, -C(NH₂)=NOH, -SO₂-NH-R₁₂, -CO-NH-R₁₂, 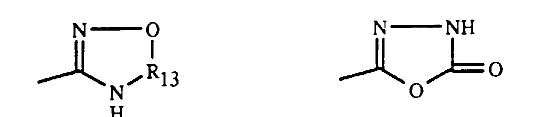 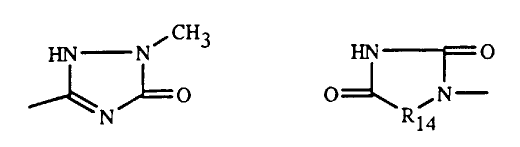 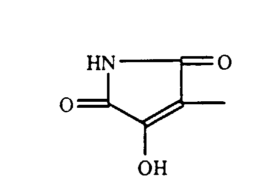 ou diméthyl-2,2 dioxo-4,6 dioxanne-1,3-yl-5.

Cette réaction s'effectue généralement au sein d'un solvant inerte tel que le tétrahydrofuranne, le diméthylformamide, un solvant chloré (chloroforme, dichloro-1,2 éthane par exemple) ou un solvant aromatique (benzène, toluène par exemple) ou un mélange de ces solvants, à une température comprise entre 20°C et la température d'ébullition du solvant.

Le dérivé réactif de l'acide carbamique peut être obtenu dans les mêmes conditions de solvant et de température.

Les dérivés de formule (Il) peuvent être obtenus par déprotection d'un dérivé de formule : 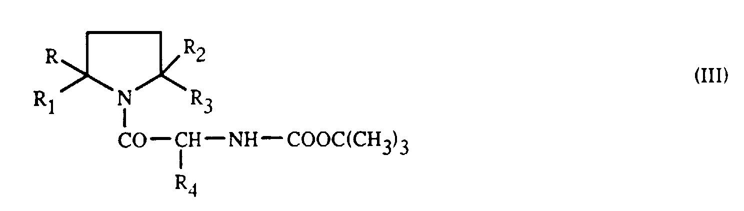 dans laquelle R, R₁, R₂, R₃ et R₄ ont les mêmes significations que dans la formule (I).

Cette déprotection s'effectue de préférence au moyen d'iodotriméthylsilane ou d'acide trifluoroacétique, au sein d'un solvant inerte tel qu'un solvant chloré (chloroforme, dichloro-1,2 éthane par exemple) ou l'acétonitrile, à une température comprise entre 15 et 40°C.

Les dérivés de formule (III) pour lesquels R₂ représente une chaîne -(CH₂)ₙ-CO-R₆ et R₆ n'est pas un radical hydroxy peuvent être obtenus par action d'un dérivé de formule : 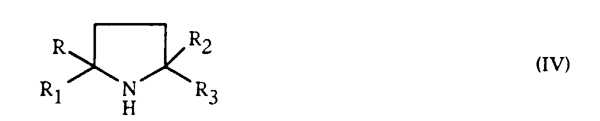 dans laquelle R, R₁ et R₃ ont les mêmes significations que dans la formule (I) et R₂ a les mêmes significations que précédemment, sur un acide de formule : 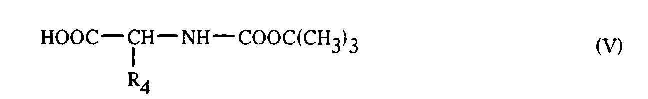 dans laquelle R₄ est défini comme dans la formule (I).

Cette réaction s'effectue au sein d'un solvant inerte tel que l'acétonitrile, le tétrahydrofuranne ou un solvant chloré, en présence d'un agent de condensation peptidique tel qu'un carbodiimide (N,N'-dicyclohexylcarbodiimide par exemple) ou un chloroformiate d'alkyle, à une température comprise entre 10 et 40°C.

Les dérivés de formule (V) peuvent être obtenus selon les méthodes habituelles de protection des amino acides.

Les dérivés de formule (IV) pour lesquels R₂ représente une chaîne -(CH₂)ₙ-CO-R₆ et n est égal à 1 ou 2 peuvent être obtenus par adaptation des méthodes décrites par S. ROSSET et coll., Tetrahedron Lett., 32, 7521 (1991); T. GALLAGHER et coll., J. Chem. Soc. Perkin Trans. I, 2193 (1991) et J. F. W. KEANA, J. Org. Chem., 48, 2644 (1983).

Les dérivés de formule (IV) pour lesquels R représente un atome d'hydrogène peuvent être obtenus par réduction des composés de formule : 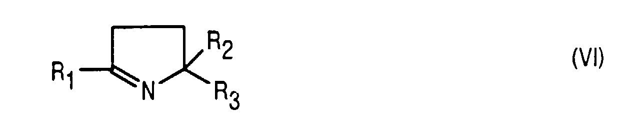 dans laquelle R₁ et R₃ ont les mêmes significations que dans la formule (I) et R₂ a les mêmes significations que dans la formule (IV).

Cette réduction s'effectue généralement au moyen d'hydrogène, en présence de charbon palladié, au sein d'un solvant inerte tel qu'un alcool ou de l'acétate d'éthyle ou au moyen de borohydrure de sodium au sein d'un alcool, en présence d'un carbonate de métal alcalin, à une température comprise entre 20°C et la température d'ébullition du milieu réactionnel.

Les dérivés de formule (VI) pour lesquels R₂ représente un radical -(CH₂)ₙ-CO-R₆, n est égal à 0, R₆ n'est pas un radical hydroxy et R₃ représente un radical alkyle, cycloalkyle ou phénylalkyle éventuellement substitué peuvent être obtenus par réaction d'un dérivé de formule (VI) correspondant pour lequel R₃ représente un atome d'hydrogène avec un dérivé de formule Hal-R₃ dans laquelle R₃ a la même signification que précédemment et Hal représente un atome d'halogène et de préférence d'iode.

Cette réaction s'effectue généralement au sein d'un solvant inerte tel que le tétrahydrofuranne ou l'éther, en présence d'une base telle que l'hydrure de sodium ou le sel de sodium ou de lithium de l'hexaméthyldisilazane, à une température comprise entre -78°C et la température d'ébullition du milieu réactionnel.

Les dérivés de formule (VI) pour lesquels R₃ représente un atome d'hydrogène et R₂ représente un radical -(CH₂)ₙ-CO-R₆, n est égal à 0, R₆ n'est pas un radical hydroxy peuvent être obtenus par déprotection et déshydratation d'un dérivé de formule : 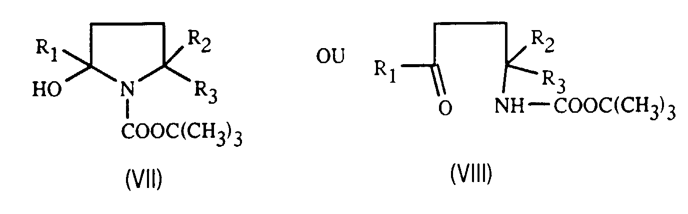 dans lesquelles R₁ a les mêmes significations que dans la formule (I), R₂ et R₃ ont les mêmes significations que précédemment, ou d'un mélange de ces dérivés.

Ces déprotection et déshydratation s'effectuent généralement au moyen d'acide trifluoroacétique ou d'iodotriméthylsilane, au sein d'un solvant inerte tel qu'un solvant chloré (dichlorométhane par exemple), à une température voisine de 20°C.

Les dérivés de formules (VII) et (VIII) peuvent être obtenus par action d'un dérivé de formule :

R₁ -M (IX)

dans laquelle R₁ a les mêmes significations que précédemment et R₁-M représente un dérivé organomagnésien, organolithien ou un cuprate, sur un dérivé de formule : 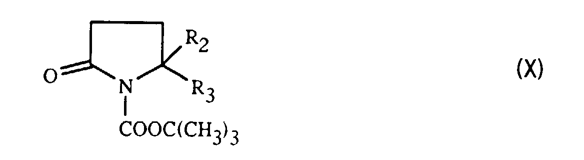 dans laquelle R₂ et R₃ ont les mêmes significations que précédemment.

Cette réaction s'effectue au sein d'un solvant inerte tel que le tétrahydrofuranne, à une température comprise entre -78°C et 20°C.

Les dérivés de formule (X) peuvent être obtenus par action de dicarbonate de ditert-butyle sur un dérivé de formule : 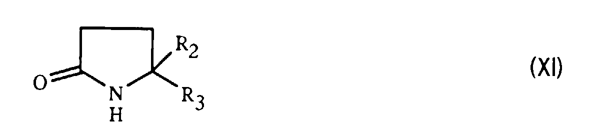 dans laquelle R₂ et R₃ ont les mêmes significations que précédemment.

Cette réaction s'effectue généralement en présence de triéthylamine ou de diméthylamino-4 pyridine, au sein d'un solvant chloré tel que le dichlorométhane, à une température voisine de 20°C.

Les dérivés de formule (XI) sont commercialisés ou peuvent être obtenus par estérification ou amidification de l'acide pyroglutamique selon les procédés décrits par M. HOLLOSI et coll., Acta Chim. (Budapest), 71, 101 (1972); B. RIGO et coll., J. Heterocycl. Chem., 25, 49 (1988); J.H. BILLMANN, J.L. RANDALL, J. Am. Chem. Soc., 66, 745 (1944); R.B. ANGIER, V.K. SMITH, J. Org. Chem., 21, 1540 (1956); J.C. SAUER, H. ADKINS, J. Am. Chem. Soc., 60, 402 (1938).

Les dérivés de formule (IV) pour lesquels R représente un radical alkyle, cycloalkyle, phénylalkyle ou phényle éventuellement substitué par un ou plusieurs substituants choisis parmi les atomes d'halogène et les radicaux alkyle et alcoxy peuvent être obtenus par réaction d'un dérivé de formule (VI) correspondant sur un dérivé de formule :

R-M (XII)

dans laquelle R a les mêmes significations que précédemment et R-M représente un organolithien ou un organomagnésien.

Cette réaction s'effectue au sein d'un solvant inerte tel que le tétrahydrofuranne ou l'éther, en présence d'un acide de Lewis tel que trifluorure de bore ou le tétrachlorure de titane, à une température comprise entre -78°C et la température d'ébullition du milieu réactionnel.

Les dérivés de formule (III) pour lesquels R₂ représente une chaîne -(CH₂)ₘ-O-CO-R"₆ peuvent être obtenus par réaction d'un dérivé de formule : 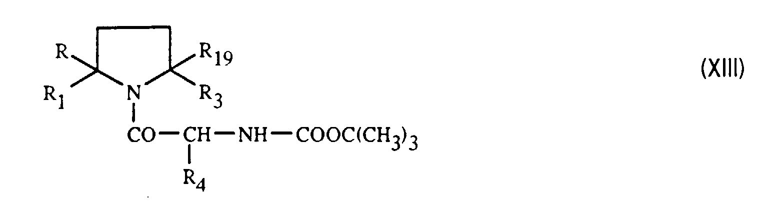 dans laquelle R, R₁, R₃ et R₄ ont les mêmes significations que dans la formule (I) et R₁₉ représente une chaîne -(CH₂)ₘ-OH, sur un halogénure de formule Hal-CO-R"₆ dans laquelle Hal représente un atome d'halogène et de préférence chlore et R"₆ a les mêmes significations que dans la formule (I).

Cette réaction s'effectue au sein d'un solvant inerte tel qu'un solvant chloré, en présence d'une trialkylamine, à une température comprise entre 20°C et la température d'ébullition du milieu réactionnel.

Les dérivés de formule (XIII) pour lesquels R₁₉ représente une chaîne -(CH₂)ₙ-OH peuvent être obtenus par réduction d'un dérivé de formule (III) correspondant pour lequel R₂ représente une chaîne -(CH₂)ₙ-CO-R₆, n est égal à 0 ou 1 et R₆ représente un radical alcoxy ou hydroxy.

Cette réaction s'effectue au sein d'un solvant inerte tel qu'un alcool (méthanol, éthanol, tertiobutanol), le tétrahydrofuranne ou un mélange de ces solvants, en présence de borohydrure de sodium ou de diborane, à une température comprise entre 20°C et la température d'ébullition du milieu réactionnel.

Les dérivés de formule (III) pour lesquels R₂ représente une chaîne -(CH₂)ₘ-O-CO-R"₆, R"₆ représente un radical -NR₉R₁₀ et R₉ représente un atome d'hydrogène peuvent également être obtenus par condensation d'un dérivé de formule (XIII) dans laquelle R₁₉ représente une chaîne -(CH₂)ₘ-OH sur un isocyanate de formule R₁₀NCO.

Cette réaction s'effectue au sein d'un solvant inerte tel qu'un solvant chloré, le tétrahydrofuranne ou le diméthylformamide, éventuellement en présence d'un quantité catalytique d'un alcoxyde de métal alcalin, à une température comprise entre 20°C et la température d'ébullition du milieu réactionnel.

Les dérivés de formule (III) pour lesquels R₂ représente un radical -(CH₂)ₘ-NR₉R₁₀ peuvent être obtenus par action d'une amine HNR₉R₁₀ dans laquelle R₉ et R₁₀ ont les mêmes significations que dans la formule (I) sur un dérivé de formule (XIII) dans laquelle R₁₉ représente un radical -(CH₂)ₘ-O-SO₂-CH₃.

Cette réaction s'effectue généralement soit en présence d'un large excès d'amine, à une température comprise entre 0 et 10°C soit lorsque l'on utilise le chlorhydrate de l'amine, au sein d'un solvant chloré, en présence d'une trialkylamine, à une température comprise entre 20°C et la température d'ébullition du milieu réactionnel.

Les dérivés de formule (XIII) dans laquelle R₁₉ représente un radical -(CH₂)ₘ-O-SO₂-CH₃ peuvent être obtenus par réaction d'un dérivé de formule (XIII) correspondant pour lequel R₁₉ représente un radical -(CH₂)ₘ-OH avec le chlorure de méthanesulfonyle.

Cette réaction s'effectue généralement au sein d'un solvant inerte tel que l'acétonitrile ou le chlorure de méthylène, en présence de triéthylamine, à une température comprise entre 0°C et la température d'ébullition du milieu réactionnel.

Les composés de formule (III) pour lesquels R₂ représente un radical -(CH₂)ₙ-CO-R₆, R₆ représente un radical hydroxy peuvent être obtenus par saponification d'un dérivé de formule (III) correspondant pour lequel R₆ représente un radical alcoxy.

Cette réaction s'effectue au sein de solvants inertes tels que le tétrahydrofuranne, le méthanol ou le dioxane et de l'eau, en présence d'une base telle que la soude, la potasse ou l'hydroxyde de lithium, à une température comprise entre 0 et 25°C.

Les dérivés de formule (III) pour lesquels R₂ représente un radical -(CH₂)ₙ-CO-R₆ et R₆ représente un radical alcoxy, cycloalcoxy ou cycloalkylalkyloxy peuvent être obtenus par estérification des dérivés de formule (III) correspondants pour lesquels R₂ représente un radical -(CH₂)ₙ-CO-R₆ et R₆ représente un radical hydroxy.

Cette réaction s'effectue de préférence au moyen d'un alcool R₁₆-OH dans lequel R₁₆ représente un radical alkyle, cycloalkyle, cycloalkylalkyle, en présence de chlorure de tosyle, au sein de la pyridine, à une température comprise entre 0 et 25°C.

Les dérivés de formule (III) pour lesquels R₂ représente un radical -(CH₂)ₙ-CO-R₆ et R₆ représente un radical phényle peuvent être obtenus par action d'un dérivé de formule (III) correspondant pour lequel R₂ représente un radical -(CH₂)ₙ-CO-R₆ et R₆ représente un radical alcoxy avec le bromure de phénylmagnésium.

Cette réaction s'effectue de préférence au sein d'un solvant inerte tel que le tétrahydrofuranne ou l'éther éthylique, à une température comprise entre -70°C et la température d'ébullition du mélange réactionnel.

Les composés de formule (III) pour lesquels R₂ représente un radical oxazolinyle éventuellement substitué peuvent être obtenus par action d'un dérivé de formule (III) correspondant pour lequel R₂ représente un radical -(CH₂)ₙ-CO-R₆, n est égal à 0 et R₆ représente un radical hydroxy, sur l'amino-2 éthanol éventuellement substitué par un ou plusieurs radicaux alkyle.

Cette réaction s'effectue au sein d'un solvant inerte tel que le toluène en éliminant l'eau formée, à la température d'ébullition du milieu réactionnel.

Les composés de formule (III) pour lesquels R₂ représente un radical alkyl-3 oxadiazolyle peuvent être obtenus par action d'un dérivé de formule (III) correspondant pour lequel R₂ représente un radical -(CH₂)ₙ-CO-R₆, n est égal à 0 et R₆ représente un radical alcoxy sur une alkylamidoxime.

Cette réaction s'effectue au sein d'un solvant inerte tel que le tétrahydrofurane, en présence d'hydrure de sodium, à une température comprise entre 25°C et la température d'ébullition du milieu réactionnel.

Les dérivés de formule (III) pour lesquels R₂ représente une chaîne -(CH₂)ₙ-CO-R₆ et R₆ représente un radical -NR₉R₁₀ peuvent être obtenus par action d'un dérivé de formule (III) correspondant pour lequel R₆ représente un radical hydroxy ou un dérivé réactif de cet acide sur une amine de formule HNR₉R₁₀.

Lorsque l'on met en oeuvre l'acide, on opère en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (diméthylformamide) ou un solvant chloré (chlorure de méthylène, dichloro-1,2 éthane, chloroforme par exemple) à une température comprise entre 0°C et la température de reflux du mélange réactionnel.

Lorsque l'on met en oeuvre un dérivé réactif de l'acide, il est possible de faire réagir l'anhydride, un anhydride mixte ou un ester (qui peut être choisi parmi les esters activés ou non de l'acide).

On opère alors soit en milieu organique, éventuellement en présence d'un accepteur d'acide tel qu'une base organique azotée (trialkylamine, pyridine, diaza-1,8 bicyclo [5.4.0] undécène-7 ou diaza-1,5 bicyclo [4.3.0] nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalinoterreux à une température comprise entre 0 et 40°C.

Les anilines éventuellement substituées sont commercialisées ou peuvent être obtenues par application ou adaptation des méthodes décrites par R. SCHRÖTER, Methoden der organischen Chemie, Houben Weil, Band XI/1, p 360; G.J. ESSELEN et coll., J. Am. Chem. Soc., 36, 322 (1914); G. ADRIANT et coll., Bull. Soc. Chim. Fr, 1511 (1970); W.A. JACOBS et coll., J. Am. Chem. Soc., 39, 2418 (1917) et J. Am. Chem. Soc., 39, 1435 (1917) et dans les exemples.

Les composés de formule (I) pour lesquels R₅ représente un radical phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène peuvent également être préparés par action d'un dérivé de formule (Il), sur un phénylisocyanate dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène.

Cette réaction s'effectue généralement au sein d'un solvant inerte tel que le tétrahydrofuranne, le diméthylformamide, un solvant chloré (chloroforme, dichloro-1,2 éthane par exemple), un solvant aromatique (benzène, toluène par exemple), à une température comprise entre 10°C et la température d'ébullition du solvant.

Les phénylisocyanates sont commercialisés ou peuvent être obtenus par application ou adaptation des méthodes décrites par R. RICHTER et coll., The Chemistry of Cyanate and their thio derivatives, S. PATAI, part 2, Wiley New York (1977) et dans les exemples.

Les composés de formule (I) pour lesquels pour lesquels R₅ représente un radical phényle éventuellement substitué, naphtyle, indolyle, quinolyle peuvent être préparés par réaction d'un dérivé de formule (Il) sur un acide de formule HOOC-R₅ dans laquelle R₅ a les mêmes significations que précédemment ou un dérivé réactif de cet acide.

Lorsque l'on met en oeuvre l'acide, on opère en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (diméthylformamide) ou un solvant chloré (chlorure de méthylène, dichloro-1,2 éthane, chloroforme par exemple) à une température comprise entre 0°C et la température de reflux du mélange réactionnel.

Lorsque l'on met en oeuvre un dérivé réactif de l'acide, il est possible de faire réagir l'anhydride, un anhydride mixte ou un ester (qui peut être choisi parmi les esters activés ou non de l'acide).

On opère alors soit en milieu organique, éventuellement en présence d'un accepteur d'acide tel qu'une base organique azotée (trialkylamine, pyridine, diaza-1,8 bicyclo [5.4.0] undécène-7 ou diaza-1,5 bicyclo [4.3.0] nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalinoterreux à une température comprise entre 0 et 40°C.

Les composés de formule (I) pour lesquels R₅ représente un radical phénylamino dont le noyau phényle est substitué par un radical carboxy, -alk-COOH, -O-alk-COOH, -alk'-COOH, -CH=CH-COOH, -CO-COOH, -S-alk-COOH, -SO-alk-COOH, -SO₂-alk-COOH, -C(=NOH)-COOH, -O-CH₂-alk'-COOH ou -CX=N-O-alk-COOH et/ou R₂ représente un chaîne -(CH₂)ₙ-COOH peuvent également être préparés par hydrolyse ou, selon le cas, hydrogénolyse des esters correspondants de formule (I).

Lorsque l'on utilise les esters d'alkyle ou de phénylalkyle, il est avantageux d'effectuer l'hydrolyse au moyen d'une base telle que la soude, la potasse ou l'hydroxyde de lithium, au sein d'un solvant inerte tel que le tétrahydrofuranne, le dioxanne, l'eau, le méthanol ou un mélange de ces solvants, à une température comprise entre 20°C et 40°C. Lorsque l'on utilise des esters de phénylalkyle, il est peut être aussi avantageux d'effectuer une hydrogénolyse au moyen d'hydrogène ou de formiate d'ammonium en présence d'un catalyseur tel que le palladium sur charbon dans un solvant tel que le méthanol ou l'acétate d'éthyle. Lorsque l'on utilise des esters de tert-butyle, il est avantageux d'effectuer l'hydrolyse au moyen d'un acide tel que l'acide trifluoroacétique.

Les composés de formule (I) pour lesquels R₅ représente un radical phénylamino dont le noyau phényle est substitué par un radical hydroxyiminoalkyle ou alcoxyiminoalkyle peuvent également être préparés par action du dérivé acylé correspondant de formule (I) sur un dérivé de formule :

H₂N-OR₁₈ (XIV)

dans laquelle R₁₈ représente un atome d'hydrogène ou un radical alkyle.

Cette réaction s'effectue généralement au sein d'un solvant inerte tel qu'un alcool (méthanol, éthanol par exemple), l'eau ou un mélange de ces solvants, à la température d'ébullition du solvant et éventuellement en présence d'une base telle que la pyridine.

Les composés de formule (I) pour lesquels R₂ représente une chaîne -(CH₂)ₙ-CO-R₆, R₆ n'étant pas un radical hydroxy et R₅ représente un radical phényle éventuellement substitué, naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène peuvent également être préparés par action d'un dérivé de formule (IV) sur un acide de formule : 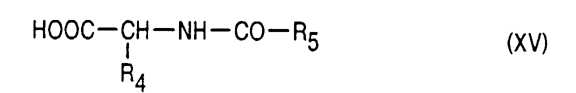 dans laquelle R₅ a les mêmes significations que ci-dessus ou un dérivé réactif de cet acide et R₄ a les mêmes significations que dans la formule (I).

Cette réaction s'effectue de préférence en présence d'un agent de condensation utilisé en chimie peptidique tel qu'un carbodiimide au sein d'un solvant tel que l'acétonitrile, le tétrahydrofuranne ou un solvant chloré ou au moyen de chlorure de thionyle dans le dichlorométhane à une température comprise entre 10°C et la température d'ébullition du solvant.

Les acides de formule (XV) peuvent être obtenus par application ou adaptation de la méthode décrite par J.R. JOHNSON et coll., J. Am. Chem. Soc., 69, 2370 (1947) ou pour les composés pour lesquels R₅ représente un radical phénylamino éventuellement substitué, par action d'un phénylisocyanate dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène sur un dérivé de formule : 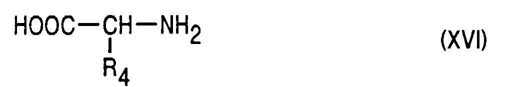 dans laquelle R₄ a les mêmes significations que dans la formule (I).

Cette réaction s'effectue généralement en solution aqueuse en présence d'une base telle qu'un bicarbonate de métal alcalin ou dans le dioxanne aqueux, à une température voisine de 20°C.

Il est entendu pour l'homme de métier que, la mise en oeuvre des procédés selon l'invention décrits précédemment, il peut être nécessaire afin d'éviter des réactions secondaires d'introduire des groupes protecteurs des fonctions amine, alcool, acide, cétone tels que ceux décrits par T. W. GREENE, protective groups in organic synthesis, John Wiley and Sons, New York. Par exemple les fonctions amine peuvent être bloquées sous forme de carbamates de tert-butyle ou de méthyle puis régénérées au moyen d'iodotriméthylsilane ou de carbamates de benzyle puis régénérées par hydrogénation après avoir mis en oeuvre le procédé selon l'invention. Les fonctions alcool peuvent par exemple être bloquées sous forme de benzoate puis régénérées par hydrolyse en milieu alcalin après avoir mis en oeuvre le procédé selon l'invention. Les fonctions cétone peuvent être bloquées sous forme de dioxolanne-1,3 puis régénérées au moyen de d'un mélange acide chlorhydrique-acide acétique.

Les énantiomères des composés de formule (I) contenant au moins un site asymétrique peuvent être obtenus par dédoublement des racémiques par exemple par chromatographie sur colonne chirale ou par synthèse à partir des précurseurs chiraux.

Les composés de formule (I) peuvent être purifiés par les méthodes connues habituelles, par exemple par cristallisation, chromatographie ou extractions.

Les composés de formule (I) comportant un reste basique peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un reste acide peuvent éventuellement être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'une amine sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les composés de formule (I) présentent des propriétés pharmacologiques intéressantes. Ces composés possèdent une forte affinité pour les récepteurs de la cholécystokinine (CCK) et de la gastrine et sont donc utiles dans le traitement et la prévention des désordres liés à la CCK et à la gastrine au niveau du système nerveux et de l'appareil gastrointestinal.

C'est ainsi que ces composés peuvent être utilisés pour le traitement ou la prévention des psychoses, des troubles anxieux, de la dépression, de la neurodégénération, des attaques de panique, de la maladie de Parkinson, de la diskynésie tardive, du syndrôme du colon irritable, de la pancréatite aiguë, des ulcères, des désordres de la motilité intestinale, de certaines tumeurs sensibles à la CCK, comme régulateur de l'appétit, dans le sevrage aux traitements chroniques et abus d'alcool ou de médicaments et comme constricteur de la pupille de l'oeil.

Ces composés ont également un effet de potentialisation sur l'activité analgésique des médicaments narcotiques et non narcotiques. En outre, ils peuvent avoir un effet analgésique propre.

Par ailleurs, les composés ayant une forte affinité pour les récepteurs CCK modifient les capacités de mémorisation. En conséquence, ces composés peuvent être efficaces dans les troubles de la mémoire.

L'affinité des composés de formule (I) pour les récepteurs CCK a été déterminée selon une technique inspirée de celle de A. SAITO et coll . (J. Neuro. Chem., 37, 483-490 (1981)) au niveau du cortex cérébral et au niveau du pancréas.

Dans ces tests, la Cl₅₀ des composés de formule (I) est généralement inférieure ou égale à 1000 nM.

Par ailleurs, il est connu que les produits qui reconnaissent les récepteurs centraux de la CCK ont une spécificité similaire pour les récepteurs de la gastrine dans le tractus gastrointestinal (BOCK et coll., J. Med. Chem., 32, 13-16 (1989); REYFELD et coll., Am. J. Physiol., 240, G255-266 (1981); BEINFELD et coll., Neuropetides, 3, 411-427 (1983).

Les composés de formule (I) présentent une toxicité faible. Leur DL₅₀ est généralement supérieure à 40 mg/kg par voie sous cutanée chez la souris.

D'un intérêt particulier sont les composés de formule (I) pour lesquels R représente un atome d'hydrogène ou un radical alkyle ou phényle, R₁ représente un radical alkyle ou phényle, R₂ représente une chaîne -(CH₂)ₙ-CO-R₆, R₃ représente un atome d'hydrogène ou un radical alkyle, R₄ représente un radical alkyle, R₅ représente un radical phénylamino dont le noyau phényle est substitué par un radical carboxy, R₆ représente un radical alcoxy et n est égal à 0, leurs sels et leurs isomères.

D'un intérêt particulier sont les composés suivants :
- acide{[(tert-butoxycarbonyl-2 diphényl-5,5 pyrrolidinyl-1)-2 oxo-2 éthyl]-3 uréido}-3 benzoïque-(S),
- acide {[(tert-butoxycarbonyl-2 méthyl-5 phényl-5 pyrrolidinyl-1)-2 oxo-2 éthyl]-3 uréido}-3 benzoïque-(2S,5R),
- acide{[(tert-butoxycarbonyl-2 méthyl-2 phényl-5 pyrrolidinyl-1)-2 oxo-2 éthyl]-3 uréido}-3 benzoïque-(2RS,5SR),
- acide{[(tert-butoxycarbonyl-2 butyl-5 pyrrolidinyl-1)-2 oxo-2 éthyl]-3 uréido}-3 benzoïque-(2S,5S),
leurs sels et leurs isomères.

Les exemples suivants illustrent l'invention sans la limiter.

### Exemple 1

A A une solution de 2 g de {[(benzyloxycarbonyl-3 phényl)-3 uréido]-2 acétyl}-1 diphényl-5,5 pyrrolidinecarboxylate-2 de tert-butyle-(S) dans 100 cm³ d'acétate d'éthyle, on ajoute 0,2 g de palladium à 10% sur charbon. La suspension est agitée pendant vingt heures à une température voisine de 20°C sous atmosphère d'hydrogène (130 kPa). Le catalyseur est séparé par filtration sur célite, rincé par 50 cm³ d'acétate d'éthyle et le filtrat est concentré à sec sous pression réduite. Le résidu est chromatographié sur silice [éluant : dichlorométhane-méthanol (90-10 en volumes)]. Les fractions contenant le produit attendu sont réunies et concentrées sous pression réduite. Le résidu est remis en suspension dans 10 cm³ d'oxyde de diisopropyle, filtré et séché sous vide à une température voisine de 40°C. On obtient ainsi 0,65 g d'acide (tert-butoxycarbonyl-2 diphényl-5,5 pyrrolidinyl-1)-2 oxo-2 éthyl]-3 uréido}-3 benzoïque-(S) fondant à 180°C; Rf= 0,22 [CCM sur silice, éluant : dichlorométhane-méthanol (90-10 en volumes)].

B Le {[(benzyloxycarbonyl-3 phényl)-3 uréido]-2 acétyl}-1 diphényl-5,5 pyrrolidinecarboxylate-2 de tert-butyle-(S) peut être obtenu de la manière suivante : à une solution de 2,8 g de diphényl-5,5 (phtalimido-2 acétyl)-1 pyrrolidinecarboxylate-2 de tert-butyle-(S) dans 50 cm³ de dichlorométhane, on ajoute, à une température voisine de 5°C, 0,9 cm³ de méthylhydrazine. Le mélange réactionnel est maintenu à une température voisine de 5°C pendant vingt heures, puis chauffé au reflux pendant quatre heures. Après refroidissement à une température voisine de 20°C, la phase organique est lavée par deux fois 50cm³ d'eau, séchée sur sulfate de magnésium et concentrée sous pression réduite. Le résidu est dilué par 50 cm³ de tétrahydrofuranne puis additioné de 1,4 g d'isocyanato-3 benzoate de benzyle. Le mélange réactionnel est agité pendant vingt heures à une température voisine de 20°C puis concentré sous pression réduite. Le résidu est purifié par chromatographie sur silice [éluant : cyclohexane-acétate d'éthyle (70-30 en volumes)]. Les fractions contenant le produit attendu sont réunies et concentrées sous pression réduite. On obtient ainsi 2 g de {[(benzyloxycarbonyl-3 phényl)-3 uréido]-2 acétyl}-1 diphényl-5,5 pyrrolidinecarboxylate-2 de tert-butyle-(S) sous forme d'une meringue utilisée telle quelle dans les synthèses ultérieures.

C Le diphényl-5,5 (phtalimido-2 acétyl)-1 pyrrolidinecarboxylate-2 de tert-butyle-(S) peut être préparé de la manière suivante : à une solution de 1 g de diphényl-5,5 pyrrolidinecarboxylate-2 de tert-butyle-(S) dans 30 cm³ de dioxanne-1,4, on ajoute, à une température voisine de 20°C, 0,25 cm³ de pyridine puis une solution de 0,7 g de chlorure de phtaloyl-glycine dans 20 cm³ de dioxanne-1,4. Le mélange réactionnel est agité pendant vingt heures à une température voisine de 20°C puis concentré sous pression réduite. Le résidu est dissous dans 100 cm³ d'acétate d'éthyle et la phase organique est lavée par deux fois 50 cm³ d'eau distillée, séchée sur sulfate de magnésium et concentrée sous pression rédiute. Le résidu est purifié par chromatographie sur silice [éluant : cyclohexane-acétate d'éthyle (70-30 en volumes)]. Les fractions contenant le produit attendu sont réunies et concentrées sous pression réduite. On obtient ainsi 1 g de diphényl-5,5 (phtalimido-2 acétyl)-1 pyrrolidinecarboxylate-2 de tert-butyle-(S) sous forme d'une meringue utilisée telle quelle dans les synthèses ultérieures.

D Le diphényl-5,5 pyrrolidinecarboxylate-2 de tert-butyle-(S) peut être préparé de la manière suivante : à une solution de 4,95 g de phényl-5 Δ5-pyrrolinecarboxylate-2 de tert-butyle-(S) dans 100 cm³ de tétrahydrofuranne on ajoute, à une température voisine de 0°C, 2,5 cm³ d'éthérate de trifluorure de bore. Le mélange est agité pendant trente minutes à une température voisine de 0°C puis on ajoute en trente minutes, à une température voisine de -70°C, 11 cm³ d'une solution 2M de phényl lithium dans un mélange éther-hexane. Le milieu réactionnel est agité pendant quatre heures à une température voisine de -50°C puis versé dans 200 cm³ d'une solution aqueuse saturée en chlorure d'ammonium. La phase aqueuse est extraite par trois fois 100 cm³ d'acétate d'éthyle. Les extraits organiques sont réunis et lavés par deux fois 100 cm³ d'eau, séchés sur sulfate de magnésium et concentrés sous pression réduite. Le résidu est purifié par chromatographie sur silice [éluant : ether de pétrole-oxyde de diéthyle (70-30 en volumes)]. Les fractions contenant le produit attendu sont réunies et concentrées sous pression réduite. On obtient ainsi 1 g de diphényl-5,5 pyrrolidinecarboxylate-2 de tert-butyle-(S) sous forme d'une huile utilisée telle quelle dans les synthèses ultérieures.

E Le phényl-5 Δ5-pyrrolinecarboxylate-2 de tert-butyle-(S) peut être préparé de la manière suivante : à une solution de 1,8 g de tert-butoxycarbonylamino-2 oxo-5 phényl-5 pentanoate de tert-butyle-(S) dans 25 cm³ de dichlorométhane, on ajoute, à une température voisine de 20°C, 2,3 cm³ d'acide trifluoroacétique. Le mélange réactionnel est agité pendant six heures à une température voisine de 20°C, puis on ajoute 120 cm³ d'une solution aqueuse saturée en hydrogénocarbonate de sodium. La phase organique est séparée par décantation, lavée par 20 cm³ d'eau distillée, séchée sur sulfate de magnésium et concentrée sous pression réduite. On obtient ainsi 0,9 g de phényl-5 Δ5-pyrrolinecarboxylate-2 de tert-butyle-(S) sous forme d'une huile utilisée telle quelle dans les synthèses ultérieures.

F Le tert-butoxycarbonylamino-2 oxo-5 phényl-5 pentanoate de tert-butyle-(S) peut être préparé de la manière suivante : à une suspension de 0,72 g de magnésium dans 20 cm³ de tétrahydrofuranne, on ajoute en trente-cinq minutes, à une température comprise entre 20 et 30°C, une solution de 2,8 cm³ de bromobenzène dans 60 cm³ de tétrahydrofuranne. Le milieu réactionnel est encore agité à une température voisine de 24°C pendant cent quarante-cinq minutes puis est additionné en vingt minutes à une solution de 5,7 g de tert-butoxycarbonyl-1 oxo-5 pyrrolidinecarboxylate-2 de tert-butyle-(S) dans 80 cm³ de tétrahydrofuranne maintenue à une température voisine de -75°C. Le milieu réactionnel est encore agité pendant trois heures à une température voisine de -78°C puis réchauffé à une température voisine de -15°C. On ajoute alors en quinze minutes, 100 cm³ d'une solution aqueuse de chlorure d'ammonium à 10%. La phase aqueuse est séparée par décantation et extraite par trois fois 100 cm³ d'oxyde de diéthyle. Les phases organiques sont réunies et lavées par deux fois 25 cm³ d'eau, séchées sur sulfate de magnésium et concentrées sous pression réduite à une température voisine de 50°C. Le résidu est purifié par cristallisation dans 20 cm³ de pentane. On obtient ainsi 2,5 g de tert-butoxycarbonylamino-2 oxo-5 phényl-5 pentanoate de tert-butyle-(S) fondant à 107°C. Ce produit peut aussi se présenter sous forme de tert-butoxycarbonyl-1 hydroxy-5 phényl-5 pyrrolidinecarboxylate-2 de tert-butyle-(2S,5RS) fondant à 85°C.

Le tert-butoxycarbonyl-1 oxo-5 pyrrolidinecarboxylate-2 de tert-butyle-(S) peut être obtenu selon la méthode décrite par J. ACKERMANN et M. MATTHES, Helv. Chim. Acta, 73, 122-132, (1990).

G L'isocyanato-3 benzoate de benzyle peut être préparé de la manière suivante : à une suspension de 2 g de charbon dans un mélange de 12,5 cm³ de chloroformiate de trichlorométhyle et de 50 cm³ de toluène, on ajoute en quinze minutes à une température voisine de -25°C, une solution d'amino-3 benzoate de benzyle dans 150 cm3 de toluène préparée en neutralisant 27 g du chlorhydrate de l'amino-3 benzoate de benzyle par 14,4 cm³ de triéthylamine dans 150 cm³ de toluène et filtrant la suspension ainsi obtenue. Le mélange réactionnel est agité à une température voisine de 25°C pendant deux heures, puis chauffé à une température voisine de 110°C pendant deux heures. Après refroidissement à une température voisine de 25°C, le milieu réactionnel est dégazé par barbottage d'azote, filtré sur papier filtre et concentré sous pression réduite à une température voisine de 52°C. On obtient ainsi 27 g d'isocyanato-3 benzoate de benzyle sous forme d'une huile utilisée telle quelle dans les synthèses ultérieures.

L'amino-3 benzoate de benzyle peut être préparé selon la méthode décrite par H.A. SHONLE et coll., J. Amer. Chem. Soc., 43, 361 (1921)

Le chlorure de phtaloyl-glycine peut être préparé selon la méthode décrite par W. GRASSMANN et E. SCHULTE-UERBING, Chem. Ber., 83, 244 (1950).

### Exemple 2

A A une solution de 0,55 g de {[(benzyloxycarbonyl-3 phényl)-3 uréido]-2 acétyl}-1 méthyl-5 phényl-5 pyrrolidinecarboxylate-2 de tert-butyle-(2S,5R) dans 100 cm³ d'acétate d'éthyle, on ajoute 0,05 g de palladium à 10% sur charbon. La suspension est agitée pendant 20 heures à une température voisine de 20°C sous atmosphère d'hydrogène (130 kPa). Le catalyseur est séparé par filtration sur célite, rincé par 50 cm³ d'acétate d'éthyle et le filtrat est concentré à sec sous pression réduite. Le résidu est chromatographié sur silice [éluant : dichlorométhane-méthanol (90-10 en volumes)]. Les fractions contenant le produit attendu sont réunies et concentrées sous pression réduite. Le résidu est remis en suspension dans 10 cm³ d'éther de pétrole, filtré et séché sous vide à une température voisine de 40°C. On obtient ainsi 0,3 g d'acide (tert-butoxycarbonyl-2 méthyl-5 phényl-5 pyrrolidinyl-1)-2 oxo-2 éthyl]-3 uréido}-3 benzoïque-(2S,5R); Rf = 0,16 [CCM sur silice, éluant : dichlorométhane-méthanol (90-10 en volumes)].

B Le {[(benzyloxycarbonyl-3 phényl)-3 uréido]-2 acétyl}-1 méthyl-5 phényl-5 pyrrolidinecarboxylate-2 de tert-butyle-(2S,5R) peut être préparé de la manière suivante : à une solution de 0,5 g de méthyl-5 phényl-5 pyrrolidinecarboxylate-2 de tert-butyle-(2S,5R) et de 0,63 g d'acide [(benzyloxycarbonyl-3 phényl)-3 uréido]-2 acétique dans 50 cm³ d'acétonitrile, on ajoute à une température voisine de 20°C, 0,4 g de N,N'-dicyclohexylcarbodiimide. Le mélange réactionnel est agité pendant vingt heures à une température voisine de 20°C, concentré sous pression réduite, repris dans 25 cm³ d'acétate d'éthyle, filtré, rincé par 5 cm³ d'acétate d'éthyle. Le filtrat est concentré sous pression réduite et le résidu purifié par chromatographie sur silice [éluant : cyclohexane-acétate d'éthyle (70-30 en volumes)]. Les fractions contenant le produit attendu sont réunies et concentrées sous pression réduite. On obtient ainsi 0,55 g de {[(benzyloxycarbonyl-3 phényl)-3 uréido]-2 acétyl}-1 méthyl-5 phényl-5 pyrrolidinecarboxylate-2 de tert-butyle-(2S,5R) sous forme d'une meringue utilisée telle quelle dans les synthèses ultérieures.

C Le méthyl-5 phényl-5 pyrrolidinecarboxylate-2 de tert-butyle-(2S,5R) peut être préparé comme décrit à l'exemple 1 D, mais à partir 4,35 g de phényl-5 Δ5-pyrrolinecarboxylate-2 de tert-butyle-(S), de 2,5 cm³ d'éthérate de trifluorure de bore et de 13,75 cm³ d'une solution 1,6 M de méthyl lithium dans l'oxyde de diéthyle, dans 100 cm³ de tétrahydrofuranne. Après traitement, on obtient 0,5 g de méthyl-5 phényl-5 pyrrolidinecarboxylate-2 de tert-butyle-(2S,5R) sous forme d'une huile utilisée telle quelle dans les synthèses ultérieures.

D L'acide [(benzyloxycarbonyl-3 phényl)-3 uréido]-2 acétique peut être préparé de la manière suivante : à une solution de 3,97 g de glycine et de 14,62 g de carbonate de potassium dans 90 cm³ d'eau, on ajoute en quinze minutes 13,4 g d'isocyanato-3 benzoate de benzyle en solution dans 70 cm³ de dioxanne-1,4. Le mélange réactionnel est agité pendant quatre heures à une température voisine de 20°C puis acidifié à pH 1 avec une solution aqueuse d'acide chlorhydrique 4N. Le produit insoluble est séparé par filtration, lavé avec trois fois 50 cm³ d'eau et séché à l'air. On obtient ainsi 13 g d'acide [(benzyloxycarbonyl-3 phényl)-3 uréido]-2 acétique utilisé tel quel dans les synthèses ultérieures.

### Exemple 3

A On opère d'une manière analogue à celle décrite à l'exemple 1 A, mais à partir de 0,54 g de {[(benzyloxycarbonyl-3 phényl)-3 uréido]-2 acétyl}-1 méthyl-2 phényl-5 pyrrolidinecarboxylate-2 de tert-butyle-(2RS,5SR) et de 0,15 g de palladium à 10% sur charbon dans 30 cm³ d'acétate d'éthyle. Après traitement, on obtient 0,25 g d'acide (tert-butoxycarbonyl-2 méthyl-2 phényl-5 pyrrolidinyl-1)-2 oxo-2 éthyl]-3 uréido}-3 benzoïque-(2RS,5SR) fondant à 150°C.

B Le {[(benzyloxycarbonyl-3 phényl)-3 uréido]-2 acétyl}-1 méthyl-2 phényl-5 pyrrolidinecarboxylate-2 de tert-butyle-(2RS,5SR) peut être préparé comme décrit à l'exemple 2 B, mais à partir de 0,80 g de méthyl-2 phényl-5 pyrrolidinecarboxylate-2 de tert-butyle-(2RS,5SR), de 1,0 g d'acide [(benzyloxycarbonyl-3 phényl)-3 uréido]-2 acétique et de 0,63 g de N,N'-dicyclohexylcarbodiimide dans 25 cm3 d'acétonitrile. Après traitement, on obtient 0,6 g de {[(benzyloxycarbonyl-3 phényl)-3 uréido]-2 acétyl}-1 méthyl-2 phényl-5 pyrrolidinecarboxylate-2 de tert-butyle-(2RS,5SR) sous forme d'une huile utilisée telle quelle dans les synthèses ultérieures.

C Le méthyl-2 phényl-5 pyrrolidinecarboxylate-2 de tert-butyle-(2RS,5SR) peut être préparé de la manière suivante : à une suspension de 5,0 g de méthyl-2 phényl-5 Δ5-pyrrolinecarboxylate-2 de tert-butyle-(RS) dans un mélange de 15 cm³ d'éthanol et de 7,5 cm³ d'eau distillée, on ajoute à une température voisine de 5°C une solution de 0,76 g de borohydrure de sodium et de 0,35 g de carbonate de sodium dans 5 cm3 d'eau distillée. Le mélange est agité pendant soixante heures à une température voisine de 20°C puis est dilué par 150 cm³ d'eau distillée. La phase aqueuse est extraite par deux fois 100 cm³ de dichlorométhane et les extraits réunis sont lavés par 50 cm³ d'eau distillée, séchés sur sulfate de magnésium et concentrés sous pression réduite. Le résidu est purifié par chromatographie sur silice [éluant : dichlorométhane puis dichlorométhane-méthanol (99-1 en volumes)]. Les fractions contenant le produit attendu sont réunies et concentrées sous pression réduite. On obtient ainsi 0,9 g de méthyl-2 phényl-5 pyrrolidinecarboxylate-2 de tert-butyle-(2RS,5SR) sous forme d'une huile utilisée telle quelle dans les synthèses ultérieures.

D Le méthyl-2 phényl-5 Δ5-pyrrolinecarboxylate-2 de tert-butyle-(RS) peut être obtenu de la manière suivante : à une suspension de 1,6 g d'hydrure de sodium à 50% dans la vaseline dans 50 cm³ de tétrahydrofuranne on ajoute goutte à goutte une solution de 7,5 g de phényl-5 Δ5-pyrrolinecarboxylate-2 de tert-butyle-(S) dans 150 cm³ de tétrahydrofuranne. Le mélange est agité pendant quatre heures à une température voisine de 25°C puis on ajoute 2,05 cm³ d'iodure de méthyle. Le milieu réactionnel est agité pendant dix-huit heures à une température voisine de 25°C puis on ajoute 5 cm³ d'eau distillée. Le mélange est concentré sous pression réduite, dilué par 200 cm³ d'une solution aqueuse de chlorure de sodium à 15%. La phase aqueuse est extraite par trois fois 100 cm³ de dichlorométhane. Les extraits organiques sont réunis et lavés par deux fois 100 cm³ d'eau distillée, séchés sur sulfate de magnésium en traitant au noir 3S et concentrés sous pression réduite. On obtient ainsi 5,1 g de méthyl-2 phényl-5 Δ5-pyrrolinecarboxylate-2 de tert-butyle-(RS) sous forme d'une huile qui se solidifie et est utilisée telle quelle dans les synthèses ultérieures.

### Exemple 4

A On opère d'une manière analogue à celle décrite à l'exemple 1 A, mais à partir de 2,4 g de {[(benzyloxycarbonyl-3 phényl)-3 uréido]-2 acétyl}-1 butyl-5 pyrrolidinecarboxylate-2 de tert-butyle-(2S,5S) et de 0,25 g de palladium à 10% sur charbon dans 100 cm³ d'éthanol. Après traitement, on obtient 1 g d'acide (tert-butoxycarbonyl-2 butyl-5 pyrrolidinyl-1)-2 oxo-2 éthyl]-3 uréido}-3 benzoïque-(2S,5S); [α]²⁰ _{D} = .38,4° (c = 1,0 ; méthanol).

B Le {[(benzyloxycarbonyl-3 phényl)-3 uréido]-2 acétyl}-1 butyl-5 pyrrolidinecarboxylate-2 de tert-butyle-(2S,5S) peut être préparé comme décrit à l'exemple 2 B, mais à partir de 1,15 g de butyl-5 pyrrolidinecarboxylate-2 de tert-butyle-(2S,5S), de 1,65 g d'acide [(benzyloxycarbonyl-3 phényl)-3 uréido]-2 acétique et de 1,05 g de N,N'-dicyclohexylcarbodiimide dans 25 cm³ d'acétonitrile. Après traitement, on obtient 2,4 g de {[(benzyloxycarbonyl-3 phényl)-3 uréido]-2 acétyl}-1 butyl-5 pyrrolidinecarboxylate-2 de tert-butyle-(2S,5S) sous forme d'une meringue utilisée telle quelle dans les synthèses ultérieures.

C Le butyl-5 pyrrolidinecarboxylate-2 de tert-butyle-(2S,5S) peut être préparé de la manière suivante : à une solution de 1,3 g de butyl-5 Δ5-pyrrolinecarboxylate-2 de tert-butyle-(S) dans 50 cm³ d'éthanol, on ajoute 0,05 g d'oxyde de platine. La suspension est agitée pendant vingt heures à une température voisine de 20°C sous atmosphère d'hydrogène (130 kPa). Le catalyseur est séparé par filtration sur célite, rincé par 50 cm³ d'éthanol et le filtrat est concentré à sec sous pression réduite. On obtient ainsi 1,2 g de butyl-5 pyrrolidinecarboxylate-2 de tert-butyle-(2S,5S) sous forme d'une huile utilisée telle quelle dans les synthèses ultérieures.

D Le butyl-5 Δ5-pyrrolinecarboxylate-2 de tert-butyle-(S) peut être préparé comme décrit à l'exemple 1 E, mais à partir de 2 g de tert-butoxycarbonylamino-2 oxo-5 nonanoate de tert-butyle-(S) et de 2,65 cm³ d'acide trifluoroacétique dans 75 cm³ de chloroforme. Après traitement, on obtient 0,6 g de butyl-5 Δ5-pyrrolinecarboxylate-2 de tert-butyle-(S) sous forme d'une huile utilisée telle quelle dans les synthèses ultérieures.

E Le tert-butoxycarbonylamino-2 oxo-5 nonanoate de tert-butyle-(S) peut être préparé de la manière suivante : à un mélange de 9,6 cm³ d'une solution 2,5 M de butyl lithium dans les hexanes et de 10 cm³ de tétrahydrofuranne, on ajoute en trente minutes, à une température voisine de -50°C, une solution de 5,71 g de tert-butoxycarbonyl-1 oxo-5 pyrrolidinecarboxylate-2 de tert-butyle-(S) dans 30 cm³ de tétrahydrofuranne. Le milieu réactionnel est encore agité pendant huit heures à une température voisine de -50°C puis réchauffé à une température voisine de 20°C et agité pendant seize heures. Le mélange est ensuite versé sur 100 cm³ d'une solution aqueuse saturée en chlorure d'ammonium. La phase aqueuse est extraite par deux fois 50 cm³ d'acétate d'éthyle. Les phases organiques sont réunies, lavées par deux fois 50 cm³ d'eau distillée, séchées sur sulfate de magnésium et concentrées sous pression réduite. Le résidu est purifié par chromatographie sur silice [éluant: cyclohexane-acétate d'éthyle (70-30 en volumes)]. Les fractions contenant le produit attendu sont réunies et concentrées sous pression réduite. On obtient ainsi 2 g de tert-butoxycarbonylamino-2 oxo-5 nonanoate de tert-butyle-(S) sous forme d'une huile utilisée telle quelle dans les synthèses ultérieures.

### Exemple 5

A On opère d'une manière analogue à celle décrite à l'exemple 1 A, mais à partir de 1,65 g de {[(benzyloxycarbonyl-3 phényl)-3 uréido]-2 acétyl}-1 isobutyl-5 pyrrolidinecarboxylate-2 de tert-butyle-(2S,5R) et de 0,25 g de palladium à 10% sur charbon dans 50 cm³ d'éthanol. Après traitement, on obtient 0,57 g d'acide (tert-butoxycarbonyl-2 isobutyl-5 pyrrolidinyl-1)-2 oxo-2 éthyl]-3 uréido}-3 benzoïque-(2S,5R) dont le pouvoir rotatoire est [α]²⁰ _{D} = -36,8° (c = 1,0; méthanol).

B Le {[(benzyloxycarbonyl-3 phényl)-3 uréido]-2 acétyl}-1 isobutyl-5 pyrrolidinecarboxylate-2 de tert-butyle-(2S,5R) peut être préparé comme décrit à l'exemple 2 B, mais à partir de 1,9 g d'isobutyl-5 pyrrolidinecarboxylate-2 de tert-butyle-(2S,5R), de 2,75 g d'acide [(benzyloxycarbonyl-3 phényl)-3 uréido]-2 acétique et de 1,73 g de N,N'-dicyclohexylcarbodiimide dans 75 cm³ d'acétonitrile. Après traitement, on obtient 1,65 g de {[(benzyloxycarbonyl-3 phényl)-3 uréido]-2 acétyl}-1 isobutyl-5 pyrrolidinecarboxylate-2 de tert-butyle-(2S,5R) sous forme d'une meringue utilisée telle quelle dans les synthèses ultérieures.

C L'isobutyl-5 pyrrolidinecarboxylate-2 de tert-butyle-(2S,5R) peut être préparé comme décrit à l'exemple 4 C, mais à partir de 1,9 g d'isobutyl-5 Δ5-pyrrolinecarboxylate-2 de tert-butyle-(S) et de 0,2 g d'oxyde de platine dans 50 cm³ d'éthanol. Après traitement, on obtient 1,9 g d'isobutyl-5 pyrrolidinecarboxylate-2 de tert-butyle-(2S,5R) sous forme d'une huile utilisée telle quelle dans les synthèses ultérieures.

D L'isobutyl-5 Δ5-pyrrolinecarboxylate-2 de tert-butyle-(S) peut être préparé comme décrit à l'exemple 1 E, mais à partir de 3 g de tert-butoxycarbonylamino-2 méthyl-7 oxo-5 octanoate de tert-butyle-(S) et de 4 cm³ d'acide trifluoroacétique dans 100 cm³ de chloroforme. Après traitement, on obtient 1,9 g d'isobutyl-5 Δ5-pyrrolinecarboxylate-2 de tert-butyle-(S) sous forme d'une huile utilisée telle quelle dans les synthèses ultérieures.

E Le tert-butoxycarbonylamino-2 méthyl-7 oxo-5 octanoate de tert-butyle-(S) peut être préparé comme décrit à l'exemple 1 F, mais à partir de 4,3 g de tert-butoxycarbonyl-1 oxo-5 pyrrolidinecarboxylate-2 de tert-butyle-(S), de 2,12 cm³ de bromure d'isobutyle et de 0,54 g de magnésium dans 90 cm³ de tétrahydrofuranne. Après traitement, on obtient 3 g de tert-butoxycarbonylamino-2 méthyl-7 oxo-5 octanoate de tert-butyle-(S) sous forme d'une huile utilisée telle quelle dans les synthèses ultérieures.

Les médicaments selon l'invention sont constitués par un composé de formule (I) sous forme libre ou sous forme d'un sel pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles dans le traitement et la prévention des désordres liés à la CCK et à la gastrine au niveau du système nerveux et de l'appareil gastrointestinal. Ces composés peuvent donc être utilisés dans le traitement et la prévention des psychoses, des troubles anxieux, de la dépression, de la neurodégénération, des attaques de panique, de la maladie de Parkinson, de la diskynésie tardive, du syndrôme du colon irritable, de la pancréatite aiguë, des ulcères, des désordres de la motilité intestinale, de certaines tumeurs sensibles à la CCK, des troubles de la mémoire, dans le sevrage aux traitements chroniques et abus d'alcool ou de médicaments, comme constricteurs de la pupille de l'oeil, comme analgésiques, comme potentialisateurs de l'activité analgésique des médicaments analgésiques narcotiques et non narcotiques et comme régulateurs de l'appétit.

Les doses dépendent de l'effet recherché, de la durée du traitement et de la voie d'administration utilisée; elles sont généralement comprises entre 0,05 g et 1 g par jour par voie orale pour un adulte avec des doses unitaires allant de 10 mg à 500 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Acide (tert-butoxycarbonyl-2 diphényl-5,5 pyrrolidinyl-1)-2 oxo-2 éthyl]-3 uréido}-3 benzoïque 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Acide (tert-butoxycarbonyl-2 méthyl-5 phényl-5 pyrrolidinyl-1)-2 oxo-2 éthyl]-3 uréido}-3 benzoïque 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Acide (tert-butoxycarbonyl-2 méthyl-5 phényl-5 pyrrolidinyl-1)-2 oxo-2 éthyl]-3 uréido}-3 benzoïque 10 mg
- Acide benzoïque 80 mg
- Alcool benzylique 0,06 cm³
- Benzoate de sodium 80 mg
- Ethanol à 95 % 0,4 cm³
- Hydroxyde de sodium 24 mg
- Propylène glycol 1,6 cm³
- Eau q.s.p. 4 cm3

## Revendications

1. Composés de formule : 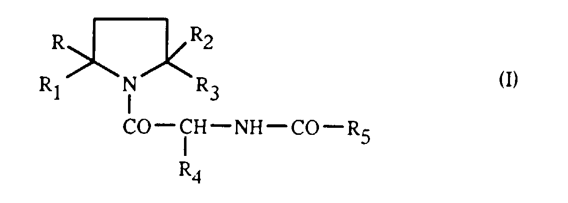 dans laquelle
R représente un atome d'hydrogène ou un radical alkyle, cycloalkyle, phénylalkyle ou phényle éventuellement substitué par un ou plusieurs substituants choisis parmi le atomes d'halogène et les radicaux alkyle et alcoxy,
R₁ représente un radical alkyle contenant 1 à 12 atomes de carbone, en chaîne droite ou ramifiée et éventuellement mono ou polyinsaturé, cycloalkyle contenant 3 à 12 atomes de carbone et éventuellement mono ou polyinsaturé, polycycloalkyle contenant 6 à 12 atomes de carbone et éventuellement mono ou polyinsaturé, phénylalkyle dont le noyau phényle est éventuellement substitué par un ou plusieurs substituants choisis parmi les radicaux alkyle, alcoxy ou les atomes d'halogène, diphénylalkyle, cinnamyle, pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₇R_{8,} -NH-CO-CH₃, trifluorométhyle ou trifluorométhoxy,
R₂ représente une chaîne -(CH₂)ₙ-CO-R₆, -(CH₂)ₘ-O-CO-R"₆, -(CH₂)ₘ-NR₉R₁₀ ou un radical oxazolinyle éventuellement substitué par un ou plusieurs radicaux alkyle ou alkyl-3 oxadiazolyle,
R₃ représente un atome d'hydrogène ou un radical alkyle, cycloalkyle ou phénylalkyle éventuellement substitué par un ou plusieurs substituants choisis parmi le atomes d'halogène et les radicaux alkyle et alcoxy,
R₄ représente un atome d'hydrogène ou un radical alkyle,
R₅ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H, sous forme de sel, -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₁, -SO₂-NH-SO₂-R₁₁, -CO-NH-CO-R₁₁, -CO-N H-SO₂-R₁₁, -B(OH)₂, -C(NH₂)=NOH, -SO₂-NH-R₁₂, -CO-NH-R₁₂, 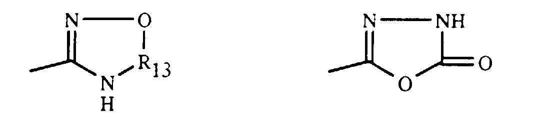 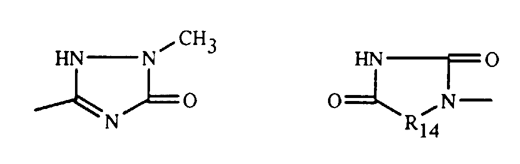 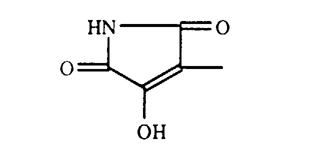 ou diméthyl-2,2 dioxo-4,6 dioxanne-1,3-yl-5,
R₆ représente un radical hydroxy, alcoxy, cycloalkyloxy, cycloalkylalkyloxy, phényle ou -NR₉R₁₀,
R"₆ représente un radical alcoxy, cycloalkyloxy, cycloalkylalkyloxy, phényle ou -NR₉R₁₀,
R₇ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
R₈ représente un radical alkyle, phénylalkyle ou phényle éventuellement substitué par un ou plusieurs substituants choisis parmi les atomes d'halogène et les radicaux alkyle, alcoxy et alkylthio,
ou bien R₇ et R₈ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,
R₉ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
R₁₀ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
ou bien R₉ et R₁₀ forment ensemble avec l'atome d'azote auquel ils sont rattachés un hétérocycle mono ou polycyclique saturé ou insaturé contenant 4 à 9 atomes de carbone et un ou plusieurs hétéroatomes (O, N, S) et éventuellement substitué par un ou plusieurs radicaux alkyle,
R₁₁ représente un radical alkyle, cycloalkyle, trifluorométhyle, phényle éventuellement substitué par un ou plusieurs substituants choisis parmi les radicaux cyano, alcoxy, nitro, amino et les atomes d'halogène,
R₁₂ représente un radical tétrazolyl-5,
R₁₃ représente C=O ou S=O,
R₁₄ représente O ou C=O,
n est égal à 0, 1 ou 2,
m est égal à 1 ou 2,
X représente un atome d'hydrogène, un radical alkyle ou phénylalkyle,
alk représente un radical alkyle ou alkylène,
alk' représente un radical hydroxyalkyle, hydroxyalkylène, alkoxyalkyle ou alkoxyalkylène
étant entendu que n est différent de 0 lorsque R et R₃ représentent chacun un atome d'hydrogène et R₁ représente un radical pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-NR₇R_{8,} -NH-CO-CH₃, trifluorométhyle ou trifluorométhoxy et étant également entendu que sauf mention contraire, les radicaux alkyle, alkylène et alcoxy et les portions alkyle, alkylène et alcoxy contiennent 1 à 4 atomes de carbone en chaîne droite ou ramifiée, les radicaux ou portions acyle contiennent 2 à 4 atomes de carbone et les radicaux et portions cycloalkyle contiennent 3 à 6 atomes de carbone ainsi que leurs sels et leurs isomères lorsqu'ils comportent au moins un centre asymétrique.

2. Composés de formule (I) selon la revendication 1 pour lesquels R₁ représente un radical isopropényle, cyclohexyle, tétrahydrophényle, cyclopentadiènyle, dihydrophényle, norbornyle, adamantyle ou norbornènyle ainsi que leurs sels et leurs isomères lorsqu'ils comportent au moins un centre asymétrique.

3. Composés de formule (I) selon la revendication 1 pour lesquels R₇ et R₈ forment avec l'atome d'azote auquel ils sont rattachés un hétérocycle choisi parmi les cycles pipéridino éventuellement substitué par un ou plusieurs radicaux alkyle ou tétrahydro-1,2,3,4 quinoléine.

4. Composés de formule (I) selon la revendication 1 pour lesquels R₉ et R₁₀ forment avec l'atome d'azote auquel ils sont rattachés un hétérocycle choisi parmi les cycles pipéridino, perhydroazépinyl-1, tétrahydro-1 ,2,3,6 pyridyl-1, tétrahydro-1,2,3,4 quinolyl-1, pyrrolidinyl-1, tétrahydro-1,2,3,4 isoquinolyl-2, thiomorpholino ou indolinyl-1, ces cycles pouvant être éventuellement substitués par au moins un radical alkyle.

5. Composés de formule (I) selon la revendication 1 pour lesquels R représente un atome d'hydrogène ou un radical alkyle ou phényle, R₁ représente un radical alkyle ou phényle, R₂ représente une chaîne -(CH₂)ₙ-CO-R₆, R₃ représente un atome d'hydrogène ou un radical alkyle, R₄ représente un radical alkyle, R₅ représente un radical phénylamino dont le noyau phényle est substitué par un radical carboxy, R₆ représente un radical alcoxy et n est égal à 0, leurs sels et leurs isomères.

6. Les composés de formule (I) selon la revendication 1 suivants :
- acide {[tert-butoxycarbonyl-2 diphényl-5,5 pyrrolidinyl-1)-2 oxo-2 éthyl]-3 uréido}-3 benzoïque-(S),
- acide {[(tert-butoxycarbonyl-2 méthyl-5 phényl-5 pyrrolidinyl-1)-2 oxo-2 éthyl]-3 uréido}-3 benzoïque-(2S,5R),
- acide {[tert-butoxycarbonyl-2 méthyl-2 phényl-5 pyrrolidinyl-1)-2 oxo-2 éthyl]-3 uréido}-3 benzoïque-(2RS,5SR),
- acide {[(tert-butoxycarbonyl-2 butyl-5 pyrrolidinyl-1)-2 oxo-2 éthyl]-3 uréido}-3 benzoïque-(2S,5S),
leurs sels et leurs isomères.

7. Procédé de préparation des composés de formule (I) selon la revendication 1 pour lesquels R₅ représente un radical phénylamino éventuellement substitué caractérisé en ce que l'on fait réagir un dérivé réactif de l'acide carbamique, obtenu éventuellement in situ par action d'un dérivé réactif de l'acide carbonique choisi parmi le N,N'-diimidazole carbonyle, le phosgène, le diphosgène, le triphosgène et le chloroformiate de p-nitrophényle sur un dérivé de formule : 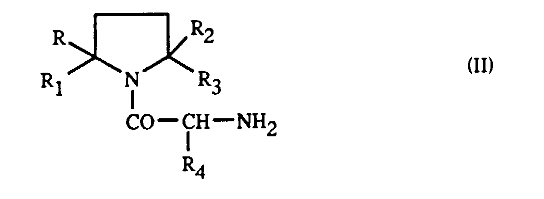 dans laquelle R, R₁, R₂, R₃ et R₄ ont les mêmes significations que dans la revendication 1, sur une aniline dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-SO₃H, sous forme de sel, -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₁, -SO₂-NH-SO₂-R₁₁, -CO-NH-CO-R₁₁, -CO-NH-SO₂-R₁₁, -B(OH)₂, -C(NH₂)=NOH, -SO₂-NH-R₁₂, -CO-NH-R₁₂, 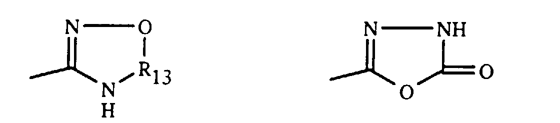 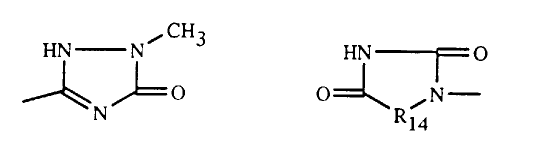 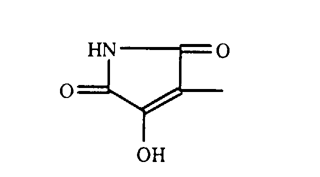 ou diméthyl-2,2 dioxo-4,6 dioxanne-1,3-yle-5, isole le produit et le transforme éventuellement en sel.

8. Procédé de préparation des composés de formule (I) selon la revendication 1 pour lesquels R₅ représente un radical phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène caractérisé en ce que l'on fait réagir un dérivé de formule : 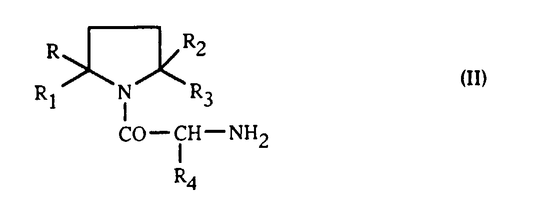 dans laquelle R, R₁, R₂, R₃ et R₄ ont les mêmes significations que dans la revendication 1, sur un phénylisocyanate dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOK, -CX=N-O-alk-COOX, -alk-COOX ou -alk'-COOX dans lesquels X est différent d'un atome d'hydrogène, isole le produit et le transforme éventuellement en sel.

9. Procédé de préparation des composés de formule (I) selon la revendication 1 pour lesquels R₅ représente un radical phényle éventuellement substitué, naphtyle, indolyle, quinolyle caractérisé en ce que l'on fait réagir un dérivé de formule : 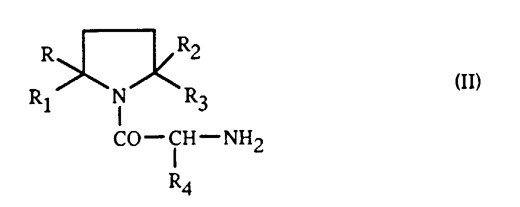 dans laquelle R, R₁, R₂, R₃ et R₄ ont les mêmes significations que dans la revendication 1, sur un acide de formule HOOC-R₅ dans laquelle R₅ a les mêmes significations que précédemment ou un dérivé réactif de cet acide, isole le produit et le transforme éventuellement en sel.

10. Procédé de préparation des composés de formule (I) selon la revendication 1 pour lesquels R₅ représente un radical phénylamino dont le noyau phényle est substitué par un radical carboxy, -alk-COOH, -O-alk-COOH, -alk'-COOH, -CH=CH-COOH, -CO-COOH, -S-alk-COOH, -SO-alk-COOH, -SO₂-alk-COOH, -C(=NOH)-COOH, -O-CH₂-alk'-COOH ou -CX=N-O-alk-COOH et/ou R₂ représente un chaîne -(CH₂)ₙ-COOH caractérisé en ce que l'on hydrolyse ou, selon le cas, hydrogénolyse un ester correspondant de formule (I), isole le produit et le transforme éventuellement en sel.

11. Procédé de préparation des composés de formule (I) selon la revendication 1 pour lesquels R₅ représente un radical phénylamino dont le noyau phényle est substitué par un radical hydroxyiminoalkyle ou alcoxyiminoalkyle caractérisé en ce que l'on fait réagir un dérivé acylé correspondant de formule (I) sur un dérivé de formule :
H₂N-OR₁₈ (XIV)
dans laquelle R₁₈ représente un atome d'hydrogène ou un radical alkyle, isole le produit et le transforme éventuellement en sel.

12. Procédé de préparation des composés de formule (I) selon la revendication 1 pour lesquels R₂ représente une chaîne -(CH₂)ₙ-CO-R₆, R₆ n'étant pas un radical hydroxy et R₅ représente un radical phényle éventuellement substitué, naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, carbamoyle, alcoxyiminoalkyle, alcoxyaminocarbonyle, -alk-O-CO-alk, -CH=CH-alk', -alk-O-alk, trifluorométhylsulfonamido, -alk-SO₃H (sous forme de sel), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX ou -alk'-COOX dans lesquels X est différent d'un atome d'hydrogène caractérisé en ce que l'on fait réagir un dérivé de formule 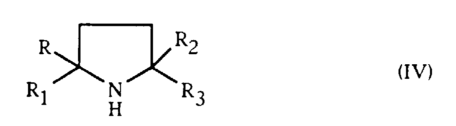 dans laquelle R, R₁ et R₃ ont les mêmes significations que dans la revendication 1 et R₂ a les mêmes significations que précédemment, sur un acide de : 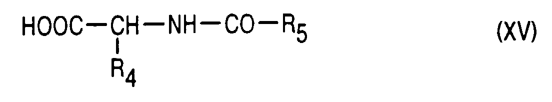 dans laquelle R₅ a les mêmes significations que ci-dessus ou un dérivé réactif de cet acide et R₄ a les mêmes significations que dans la revendication 1, isole le produit et le transforme éventuellement en sel.

13. Médicaments caractérisés en ce qu'ils contiennent comme principe actif au moins un des composés selon l'une des revendications 1 à 6.

14. Médicaments selon la revendication 13 utilisables pour le traitement des désordres liés à la CCK et à la gastrine.

## Patentansprüche

1. Verbindungen der Formel (I) 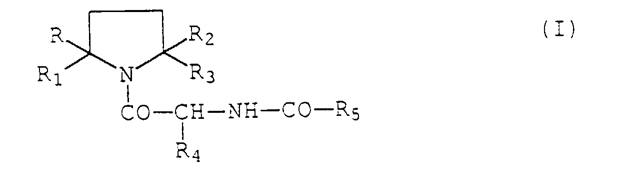 in der
R ein Wasserstoffatom oder einen Rest Alkyl, Cycloalkyl, Phenylalkyl oder Phenyl, gegebenenfalls substituiert durch einen oder mehrere Substituenten, ausgewählt unter den Halogenatomen und den Resten Alkyl und Alkoxy, darstellt,
R₁ einen Alkylrest mit 1 bis 12 Kohlenstoffatomen in gerader oder verzweigter Kette und gegebenenfalls mono- oder polyungesättigt, Cycloalkyl mit 3 bis 12 Kohlenstoffatomen und gegebenenfalls mono- oder polyungesättigt, Polycycloalkyl mit 6 bis 12 Kohlenstoffatomen und gegebenenfalls mono- oder polyungesättigt, Phenylalkyl, dessen Phenylkern gegebenenfalls substituiert ist durch einen oder mehrere Substituenten, ausgewählt unter den Resten Alkyl, Alkoxy oder den Halogenatomen, Diphenylalkyl, Cinnamyl, Pyridyl, gegebenenfalls substituiert durch einen oder mehrere Alkylreste, Furyl, gegebenenfalls substituiert durch einen oder mehrere Alkylreste, Thienyl, gegebenenfalls substituiert durch einen oder mehrere Alkylreste, Chinolyl, gegebenenfalls substituiert durch einen oder mehrere Alkylreste, Naphthyl, gegebenenfalls substituiert durch einen oder mehrere Alkylreste, Indolyl, gegebenenfalls substituiert durcn einen oder mehrere Alkylreste oder Phenyl, gegebenenfalls substituiert durch einen oder mehrere Substituenten, ausgewählt unter den Halogenatomen und den Resten Alkyl, Alkoxy, Hydroxy, Nitro, Amino, Monoalkylamino, Dialkylamino, Alkoxycarbonyl, -CO-NR₇R₈, -NH-CO-CH₃, Trifluormethyl oder Trifluormethoxy darstellt,
R₂ eine Kette -(CH₂)ₙ-CO-R₅, -(CH₂)ₘ-O-CO-R"₆, -(CH₂)ₘ-NR₉R₁₀ oder einen Oxazolinylrest, gegebenenfalls substituiert durch einen oder mehrere Alkylreste oder 3-Alkyl-oxadiazolyl darstellt,
R₃ ein Wasserstoffatom oder einen Rest Alkyl, Cycloalkyl oder Phenylalkyl bedeutet, gegebenenfalls substituiert durch einen oder mehrere Substituenten, ausgewählt unter den Halogenatomen und den Resten Alkyl und Alkoxy,
R₄ ein Wasserstoffatom oder einen Alkylrest bedeutet,
R₅ einen Phenylrest (gegebenenfalls substituiert durch einen oder mehrere Substituenten, ausgewählt unter den Halogenatomen und den Resten Alkyl, Alkoxy und Alkylthio), Naphthyl, Indolyl, Chinolyl oder Phenylamino, dessen Phenylkern gegebenenfalls substituiert ist durch einen oder mehrere Substituenten, ausgewählt unter den Halogenatomen und den Resten Alkyl, Alkoxy, Alkylthio, Trifluormethyl, Carboxy, Alkoxycarbonyl, Hydroxy, Nitro, Amino, Acyl, Cyano, Sulfamoyl, Carbamoyl, Hydroxyiminoalkyl, Alkoxyiminoalkyl, Hydroxyaminocarbonyl, Alkoxyaminocarbonyl, 5-Tetrazolyl, 5-Tetrazolylalkyl, Trifluormethylsulfonamido, Alkylsulfinyl, Mono- oder Polyhydroxyalkyl, Sulfo, -alk-O-CO-alk, -alk-COOX, -alk-O-alk, -alk'-COOX, -O-alk-COOX, -CH=CH-CCOX, -CO-COOX, -alk-SO₃H, in Form von Salz, -CH=CH-alk', -C(=NOH)-COOX, -S-alk-COOX, -SO-alk-COOX, -SO₂-alk-COOX, -O-CH₂-alk'-COOX, -CX=N-O-alk-COOX,
-alk-N(OH)-CO-alk, -alk-SO₂H, -SO₂-NH-CO-R₁₁, -SO₂-NH-SO₂-R₁₁, -CO-NH-CO-R₁₁, -CO-NH-SO₂-R₁₁, -B(OH)₂, -C(NH₂)=NOH, -SO₂-NH-R₁₂, -CO-NH-R₁₂, 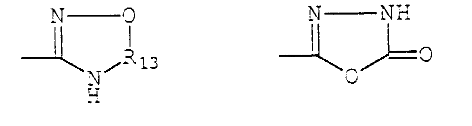 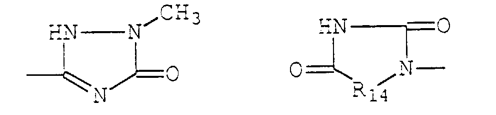 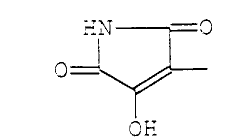 oder 2,2-Dimethyl-4,6-dioxo-1,3-dioxan-5-yl darstellt,
R₆ einen Rest Hydroxy, Alkoxy, Cycloalkyloxy, Cycloalkylalkyloxy, Phenyl oder -NR₉R₁₀ bedeutet,
R"₆ einen Rest Alkoxy, Cycloalkyloxy, Cycloalkylalkyloxy, Phenyl oder -NR₉R₁₀ darstellt,
R₇ ein Wasserstoffatom oder einen Rest Alkyl, Phenylalkyl oder Phenyl, gegebenenfalls substituiert durch einen oder mehrere Substituenten, ausgewählt unter den Halogenatomen und den Resten Alkyl, Alkoxy und Alkylthio, bedeutet,
R₈ einen Rest Alkyl, Phenylalkyl oder Phenyl, gegebenenfalls substituiert durch einen oder mehrere Substituenten, ausgewählt unter den Halogenatomen und den Resten Alkyl, Alkoxy und Alkylthio, darstellt,
oder R₇ und R₉ zusammen mit dem Stickstoffatom, an das sie gebunden sind, einen mono- oder polycyclischen, gesättigten oder ungesättigten Heterocyclus mit 4 bis 9 Kohlenstoffatomen und einem oder mehreren Heteroatomen (O, N) und gegebenenfalls substituiert durch einen oder mehrere Alkylreste, bilden,
R₉ ein Wasserstoffatom oder einen Rest Alkyl, Cycloalkylalkyl, Cycloalkyl, Phenylalkyl, oder Phenyl, gegebenenfalls substituiert durch einen oder mehrere Substituenten, ausgewählt unter den Halogenatomen und den Resten Alkyl, Alkoxy und Alkylthio, bedeutet, R₁₀ einen Rest Alkyl, Cycloalkylalkyl, Cycloalkyl, Phenylalkyl, oder Phenyl, gegebenenfalls substituiert durch einen oder mehrere Substituenten, ausgewählt unter den Halogenatomen und den Resten Alkyl, Alkoxy und Alkylthio, darstellt,
oder R₉ und R₁₀ zusammen mit dem Stickstoffatom, an das sie gebunden sind, einen mono- oder polycyclischen, gesättigten oder ungesättigten Heterocyclus mit 4 bis 9 Kohlenstoffatomen und einem oder mehreren Heteroatomen (O, N, S) und gegebenenfalls substituiert durch einen oder mehrere Alkylreste, bilden,
R₁₁ einen Rest Alkyl, Cycloalkyl, Trifluormethyl, Phenyl, gegebenenfalls substituiert durch einen oder mehrere Substituenten, ausgewählt unter den Resten Cyano, Alkoxy, Nicro, Amino und den Halogenatomen, bedeutet,
R₁₂ einen 5-Tetrazolylrest darstellt,
R₁₃ C=O oder S=O bedeutet,
R₁₄ O oder C=O bedeutet,
n gleich 0, 1 oder 2 ist,
m gleich 1 oder 2 ist,
X ein Wasserstoffatom, einen Rest Alkyl oder Phenylalkyl darstellt,
alk einen Rest Alkyl oder Alkylen bedeutet,
alk' einen Rest Hydroxyalkyl, Hydroxyalkylen, Alkoxyalkyl oder Alkoxyalkylen bedeutet,
mit der Maßgabe, daß n von 0 verschieden ist, wenn R und R₃ jeweils ein Wasserstoffatom darstellen und R₁ einen Rest Pyridyl, gegebenenfalls substituiert durch einen oder mehrere Alkylreste, Furyl, gegebenenfalls substituiert durch einen oder mehrere Alkylreste, Thienyl, gegebenenfalls substituiert durch einen oder mehrere Alkylreste, Chinolyl, gegebenenfalls substituiert durch einen oder mehrere Alkylreste, Naphthyl, gegebenenfalls substituiert durch einen oder mehrere Alkylreste, Indolyl, gegebenenfalls substituiert durch einen oder mehrere Alkylreste oder Phenyl, gegebenenfalls substituiert durch einen oder mehrere Substituenten, ausgewählt unter den Halogenatomen und den Resten Alkyl, Alkoxy, Hydroxy, Nitro, Amino, Monoalkylamino, Dialkylamino, Alkoxycarbonyl, -CO-NR₇R₉, -NH-CO-CH₃, Trifluormethyl oder Trifluormethoxy bedeutet, und mit der Maßgabe, daß außer gegenteiliger Erwähnung die Reste Alkyl, Alkylen und Alkoxy und die Teile Alkyl, Alkylen und Alkoxy 1 bis 4 Kohlenstoffatome in gerader oder verzweigter Kette, die Reste oder Teile Acyl 2 bis 4 Kohlenstoffatome und die Reste und Teile Cycloalkyl 3 bis 6 Kohlenstoffatome enthalten, sowie ihre Salze und ihre Isomere, wenn sie mindestens ein asymmetrisches Zentrum umfassen.

2. Verbindungen der Formel (I) nach Anspruch 1, worin R₁ einen Rest Isopropenyl, Cyclohexyl, Tetrahydrophenyl, Cyclopentadienyl, Dihydrophenyl, Norbornyl, Adamantyl oder Norbornenyl darstellt, sowie ihre Salze und ihre Isomere, wenn sie mindestens ein asymmetrisches Zentrum umfassen.

3. Verbindungen der Formel (I) nach Anspruch 1, worin R₇ und R₈ zusammen mit dem Stickstoffatom, an das sie gebunden sind, einen Heterocyclus bilden, ausgewählt unter den Ringen Piperidino, gegebenenfalls substituiert durch einen oder mehrere Alkylreste, oder 1,2,3,4-Tetrahydrochinolin.

4. Verbindungen der Formel (I) nach Anspruch 1, worin R₉ und R₁₀ zusammen mit dem Stickstoffatom, an das sie gebunden sind, einen Heterocyclus bilden, ausgewählt unter den Ringen Piperidino, 1-Perhydroazepinyl, 1,2,3,6-Tetrahydro-1-pyridyl, 1,2,3,4-Tetrahydro-1-chinolyl, 1-Pyrrolidinyl, 1,2,3,4-Tetrahydro-2-isochinolyl, Thiomorpholino oder 1-Indolinyl, wobei diese Ringe gegebenenfalls durch einen oder mehrere Alkylreste substituiert sein können.

5. Verbindungen der Formel (I) nach Anspruch 1, worin R ein Wasserstoffatom oder einen Rest Alkyl oder Phenyl darstellt, R₁ einen Rest Alkyl oder Phenyl bedeutet, R₂ eine Kette -(CH₂)ₙ-CO-R₆ ist, R₃ ein Wasserstoffatom oder einen Alkylrest darstellt, R₄ einen Alkylrest bedeutet, R₅ einen Phenylaminorest darstellt, dessen Phenylkern durch einen Carboxyrest substituiert ist, R₅ einen Alkoxyrest bedeutet und n gleich 0 ist, sowie ihre Salze und ihre Isomere.

6. Die folgenden Verbindungen der Formel (I) nach Anspruch 1:
- 3-{3-[2-(2-Tert.-butoxycarbonyl-5,5-diphenyl-1-pyrrolidinyl)-2-oxo-ethyl]-ureido}-(S)benzoesäure,
- 3-{3-[2-(2-Tert.-butoxycarbonyl-5-methyl-5-phenyl-1-pyrrolidinyl)-2-oxo-ethyl]-ureido}- (2S,5R)benzoesäure,
- 3-{3-[2-(2-Tert.-butoxycarbonyl-2-methyl-5-phenyl-1-pyrrolidinyl)-2-oxo-ethyl]-ureido}-(2RS,5SR)benzoesäure,
- 3-{3-[2-(2-Tert.-butoxycarbonyl-5-butyl-1-pyrrolidinyl)-2-oxo-ethyl]-ureido}-(2S,5S)benzoesäure,
sowie ihre Salze und ihre Isomere.

7. Verfahren zur Herstellung der Verbindungen der Formel (I) nach Anspruch 1, worin R₅ einen gegebenenfalls substituierten Phenylaminorest darstellt, dadurch gekennzeichnet, daß man ein reaktives Derivat der Carbaminsäure, gegebenenfalls in situ erhalten durch Umsetzung eines reaktiven Derivates der Carbonsäure, ausgewâhlt unter N,N'-Carbonyldiimidazol, Phosgen, Diphosgen, Triphosgen und Chlorameisensäure-p-nitrophenylescter, mit einem Derivat der Formel (II) 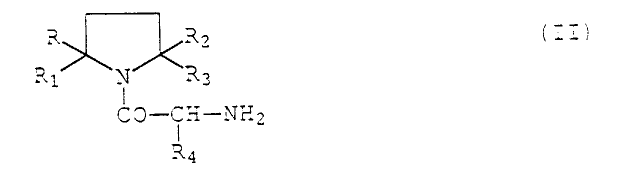 in der R, R₁, R₂, R₃ und R₄ die gleichen Bedeutungen wie in Anspruch 1 besitzen, mit einem Anilin zur Reaktion bringt, dessen Phenylkern gegebenenfalls durch einen oder mehrere Substituenten substituiert ist, ausgewählt unter den Halogenatomen und den Resten Alkyl, Alkoxy, Alkylthio, Trifluormethyl, Carboxy, Alkoxycarbonyl, Hydroxy, Nitro, Amino, Acyl, Cyano, Sulfamoyl, Carbamoyl, Hydroxyiminoalkyl, Alkoxyiminoalkyl, Hydroxyaminocarbonyl, Alkoxyaminocarbonyl, 5-Tetrazolyl, 5-Tetrazolylalkyl, Trifluormethylsulfonamido, Alkylsulfinyl, Mono- oder Polyhydroxyalkyl, Sulfo, -alk-O-CO-alk, -alk-COOX, -alk-O-alk, -alk'-COOX, -O-alk-COOX, -CH=CH-COOX, -CO-COOX, -alk-SO₃H, in Form von Salz, -CH=CH-alk', -C(=NOH)-COOX, -S-alk-COOX, -SO-alk-COOX, -SO₂-alk-COOX, -O-CH₂-alk'-COOX, -CX=N-O-alk-COOX, -alk-N(OH)-CO-alk, -alk-SO₂H, -SO₂-NH-CO-R₁₁, -SO₂-NH-SO₂-R₁₁, -CO-NH-CO-R₁₁, -CO-NH-SO₂-R₁₁, -B(OH)₂, -C(NH₂)=NOH, -SO₂-NH-R₁₂, -CO-NH-R₁₂, 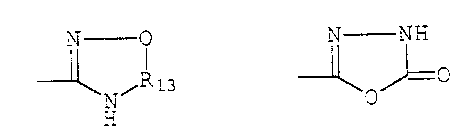 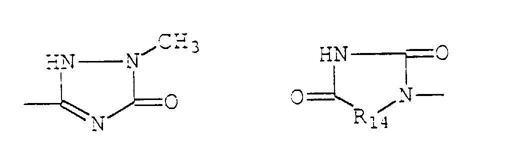 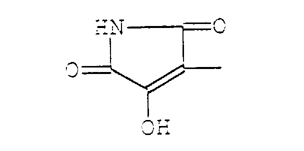 oder 2,2-Dimethyl-4,6-dioxo-1,3-dioxan-5-yl darstellt, das Produkt isoliert und gegebenenfalls in Salz überführt.

8. Verfahren zur Herstellung der Verbindungen der Formel (I) nach Anspruch 1, worin R₅ einen Phenylaminorest darstellt, dessen Phenylkern gegebenenfalls durch einen oder mehrere Substituenten substituiert ist, ausgewählt unter den Halogenatomen und den Resten Alkyl, Alkoxy, Alkylthio, Trifluormethyl, Nitro, Acyl, Cyano, Sulfamoyl, Alkoxycarbonyl, Carbamoyl, Alkoxyiminoalkyl, Alkoxyaminocarbonyl, -alk-O-CO-alk, -CH=CH-alk', -alk-O-alk, Trifluormethylsulfonamido, -alk-SO₃H (in Form von Salz), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX oder -alk'-COOX, worin X von einem Wasserstoffatom verschieden ist, dadurch gekennzeichnet, daß man ein Derivat der Formel (II) 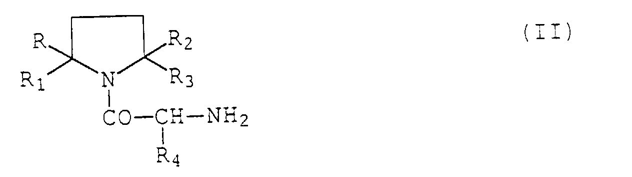 in der R, R₁, R₂, R₃ und R₄ die gleichen Bedeutungen wie in Anspruch 1 besitzen, mit einem Phenylisocyanat zur Reaktion bringt, dessen Phenylkern gegebenenfalls durch einen oder mehrere Substituenten substituiert ist, ausgewählt unter den Halogenatomen und den Resten Alkyl, Alkoxy, Alkylthio, Trifluormethyl, Nitro, Acyl, Cyano, Sulfamoyl, Alkoxycarbonyl, Carbamoyl, Alkoxyiminoalkyl, Alkoxyaminocarbonyl, -alk-O-CO-alk, -CH=CH-alk', -alk-O-alk, Trifluormethylsulfonamido, -alk-SO₃H (in Form von Salz), -O-alk-COOX, -CH=CH-COOX, -CO-COOX, -S-alk-COOX, -SO-alk-COOX, -SO₂-alk-COOX, -O-CH₂.- alk'-COOX, -CX=N-O-alk-COOX, -alk-COOX oder -al'-COOX, worin X von einem Wasserstoffatom verschieden ist, das Produkt isoliert und gegebenenfalls in Salz überführt.

9. Verfahren zur Herstellung der Verbindungen der Formel (I) nach Anspruch 1, worin R₅ einen Rest Phenyl, gegebenenfalls substituiert, Naphthyl, Indolyl oder Chinolyl darstellt, dadurch gekennzeichnet, daß man ein Derivat der Formel (II) 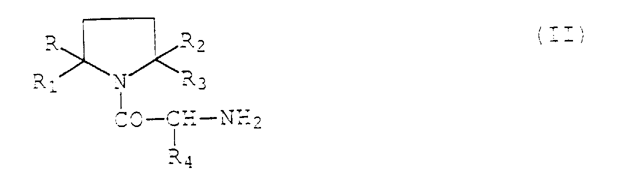 in der R, R₁, R₂, R₃ und R₄ die gleichen Bedeutungen wie in Anspruch 1 besitzen, mit einer Säure der Formel HOOC-R₅ oder mit einem reaktiven Derivat dieser Säure zur Reaktion bringt, worin R₅ die gleichen Bedeutungen wie oben besitzt, das Produkt isoliert und gegebenenfalls in Salz überführt.

10. Verfahren zur Herstellung der Verbindungen der Formel (I) nach Anspruch 1, worin R₅ einen Phenylaminorest darstellt, dessen Phenylkern durch einen Rest Carboxy, -alk-COOH, -O-alk-COOH, -alk'-COOH, -CH=CH-COOH, -CO-COOH, -S-alk-COOH, -SO-alk-COOH, -SO₂-alk-COOH, -C(=NOH) -COOH, -O-CH₂-alk'-COOH oder -CX=N-O-alk-COOH, substituiert ist, und/oder R₂ eine Kette -(CH₂)ₙ-COOH bedeutet, dadurch gekennzeichnet, daß man einen entsprechenden Ester der Formel (I) hydrolysiert oder gegebenenfalls hydrogenolysiert, das Produkt isoliert und gegebenenfalls in Salz überführt.

11. Verfahren zur Herstellung der Verbindungen der Formel I, nach Anspruch 1, worin R₅ einen Phenylaminorest darstellt, dessen Phenylkern durch einen Rest Hydroxyiminoalkyl oder Alkoxyiminoalkyl substituiert ist, dadurch gekennzeichnet, daß man ein entsprechendes Acylderivat der Formel (I) mit einem Derivat der Formel (XIV)
H₂N-OR₈ (XIV)
in der R₁₈ ein Wasserstoffatom oder einen Alkylrest darstellt, zur Reaktion bringt, das Produkt isoliert und gegebenenfalls in Salz überführt.

12. Verfahren zur Herstellung der Verbindungen der Formel (I) nach Anspruch 1, worin R₂ eine Kette -(CH₂)ₙ-CO-R₆ darstellt, R₆ kein Hydroxyrest ist und R₅ einen Rest Phenyl, gegebenenfalls substituiert, Naphthyl, Indolyl, Chinolyl oder Phenylamino darstellt, dessen Phenylkern gegebenenfalls durch einen oder mehrere Substituenten substituiert ist, ausgewählt unter den Halogenatomen und den Resten Alkyl, Alkoxy, Alkylthio, Trifluormethyl, Nitro, Acyl, Cyano, Sulfamoyl, Alkoxycarbonyl, Carbamoyl, Alkoxyiminoalkyl, Alkoxyaminocarbonyl, -alk-O-CO-alk, -CH=CH-alk', -alk-O-alk, Trifluormethylsulfonamido, -alk-SO₃H (in Form von Salz) -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX oder -alk'-COOX, worin X von einem Wasserstoffatom verschieden ist, dadurch gekennzeichnet, daß man ein Derivat der Formel (IV) 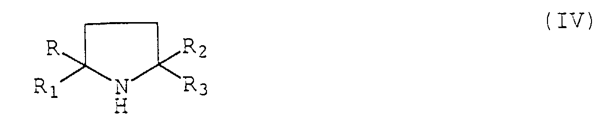 in der R, R₁ und R₃ die gleichen Bedeutungen wie in Anspruch 1 besitzen und R₂ die gleiche Bedeutung wie vorstehend aufweist, mit einer Säure der Formel (XV) 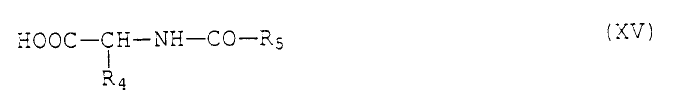 oder mit einem reaktiven Derivat dieser Säure zur Reaktion bringt, worin R₅ die gleiche Bedeutung wie oben und R₄ die gleiche Bedeutung wie in Anspruch 1 besitzt, das Produkt isoliert und gegebenenfalls in Salz überführt.

13. Arzneimittel, dadurch gekennzeichnet, daß sie als Wirkstoff mindestens eine der Verbindungen nach einem der Ansprüche 1 bis 6 enthalten.

14. Arzneimittel nach Anspruch 13 , anwendbar zur Behandlung von Störungen, die mit CCK und Gastrin in Zusammenhang stehen.

## Claims

1. Compounds of formula: 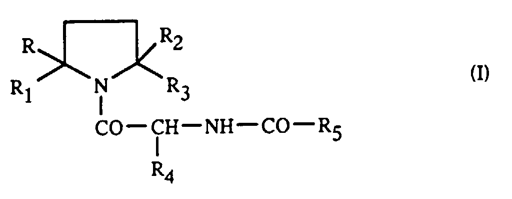 in which
R represents a hydrogen atom or an alkyl, cycloalkyl or phenylalkyl radical or a phenyl radical optionally substituted by one or more substituents chosen from halogen atoms and alkyl and alkoxy radicals,
R₁ represents an alkyl radical containing 1 to 12 carbon atoms in a straight or branched chain and optionally mono- or polyunsaturated, a cycloalkyl radical containing 3 to 12 carbon atoms and optionally mono- or polyunsaturated, a polycycloalkyl radical containing 6 to 12 carbon atoms and optionally mono- or polyunsaturated, a phenylalkyl radical in which the phenyl ring is optionally substituted by one or more substituents chosen from alkyl or alkoxy radicals or halogen atoms, a diphenylalkyl radical, a cinnamyl radical, a pyridyl radical optionally substituted by one or more alkyl radicals, a furyl radical optionally substituted by one or more alkyl radicals, a thienyl radical optionally substituted by one or more alkyl radicals, a quinolyl radical optionally substituted by one or more alkyl radicals, a naphthyl radical optionally substituted by one or more alkyl radicals, an indolyl radical optionally substituted by one or more alkyl radicals or a phenyl radical optionally substituted by one or more substituents chosen from halogen atoms and alkyl, alkoxy, hydroxyl, nitro, amino, monoalkylamino, dialkylamino, alkoxycarbonyl, -CO-NR₇R₈, -NH-CO-CH₃, trifluoromethyl or 5 trifluoromethoxy radicals,
R₂ represents a -(CH₂)ₙ-CO-R₆, -(CH₂)ₘ-O-CO-R"₆ or -(CH₂)ₘ-NR₉R₁₀ chain, an oxazolinyl radical optionally substituted by one or more alkyl radicals or a 3-alkyloxadiazolyl radical,
R₃ represents a hydrogen atom or an alkyl or cycloalkyl radical or a phenylalkyl radical optionally substituted with one or more substituents chosen from halogen atoms and alkyl and alkoxy radicals,
R₄ represents a hydrogen atom or an alkyl radical,
R₅ represents a phenyl radical (optionally substituted by one or more substituents chosen from halogen atoms and alkyl, alkoxy and alkylthio radicals), a naphthyl radical, an indolyl radical, a quinolyl radical or a phenylamino radical in which the phenyl ring is optionally substituted by one or more substituents chosen from halogen atoms and alkyl, alkoxy, alkylthio, trifluoromethyl, carboxyl, alkoxycarbonyl, hydroxyl, nitro, amino, acyl, cyano, sulphamoyl, carbamoyl, hydroxyiminoalkyl, alkoxyiminoalkyl, hydroxyaminocarbonyl, alkoxyaminocarbonyl, 5-tetrazolyl, 5-tetrazolylalkyl, trifluoromethylsulphonamido, alkylsulphinyl, mono- or polyhydroxyalkyl, sulpho, -alk-O-CO-alk, -alk-COOX, -alk-O-alk, -alk'-COOX, -O-alk-COOX, -CH=CH-COOX, -CO-COOX, -alk-SO₃H in salt form, -CH=CH-alk', -C(=NOH)-COOX, -S-alk-COOX, -SO-alk-COOX, -SO₂-alk-COOK, -O-CH₂-alk'-COOX, -CX=N-O-alk-COOX, -alk-N(OH)-CO-alk, -alk-SO₂H, -SO₂-NH-CO-R₁₁, -SO₂-NH-SO₂-R₁₁, -CO-NH-CO-R₁₁, -CO-NH-SO₂-R₁₁, -B(OH)₂, -C(NH₂)=NOH, -SO₂-NH-R₁₂, -CO-NH-R₁₂, 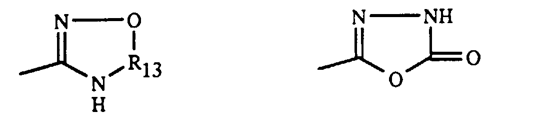 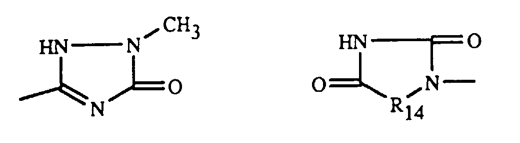 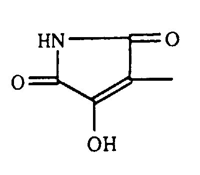 or 2,2-dimethyl-4,6-dioxo-1,3-dioxan-5-yl radicals,
R₆ represents a hydroxyl, alkoxy, cycloalkyloxy, cycloalkylalkyloxy, phenyl or -NR₉R₁₀ radical,
R"₆ represents an alkoxy, cycloalkyloxy, cycloalkylalkyloxy, phenyl or -NR₉R₁₀ radical,
R₇ represents a hydrogen atom or an alkyl radical, a phenylalkyl radical or a phenyl radical optionally substituted by one or more substituents chosen from halogen atoms and alkyl, alkoxy and alkylthio radicals, R₈ represents an alkyl radical, a phenylalkyl radical or a phenyl radical optionally substituted by one or more substituents chosen from halogen atoms and alkyl, alkoxy and alkylthio radicals,
or else R₇ and R₈ form, with the nitrogen atom to which they are attached, a saturated or unsaturated, mono- or polycyclic heterocycle containing 4 to 9 carbon atoms and one or more hetero atoms (O, N) and optionally substituted by one or more alkyl radicals,
R₉ represents a hydrogen atom or an alkyl radical, a cycloalkylalkyl radical, a cycloalkyl radical, a phenylalkyl radical or a phenyl radical optionally substituted by one or more substituents chosen from halogen atoms and alkyl, alkoxy and alkylthio radicals,
R₁₀ represents an alkyl radical, a cycloalkylalkyl radical, a cycloalkyl radical, a phenylalkyl radical or a phenyl radical optionally substituted by one or more substituents chosen from halogen atoms and alkyl, alkoxy and alkylthio radicals,
or else R₉ and R₁₀ form, together with the nitrogen atom to which they are attached, a saturated or unsaturated, mono- or polycyclic heterocycle containing 4 to 9 carbon atoms and one or more hetero atoms (O, N, S) and optionally substituted by one or more alkyl radicals,
R₁₁ represents an alkyl radical, a cycloalkyl radical, a trifluoromethyl radical or a phenyl radical optionally substituted by one or more substituents chosen from cyano, alkoxy, nitro or amino radicals and halogen atoms,
R₁₂ represents a 5-tetrazolyl radical,
R₁₃ represents C=O or S=O,
R₁₄ represents O or C=O,
n is equal to 0, 1 or 2,
m is equal to 1 or 2,
X represents a hydrogen atom or an alkyl or phenylalkyl radical,
alk represents an alkyl or alkylene radical,
alk' represents a hydroxyalkyl, hydroxyalkylene, alkoxyalkyl or alkoxyalkylene radical,
it being understood that n is other than 0 when R and R₃ each represent a hydrogen atom and R₁ represents a pyridyl radical optionally substituted by one or more alkyl radicals, a furyl radical optionally substituted by one or more alkyl radicals, a thienyl radical optionally substituted by one or more alkyl radicals, a quinolyl radical optionally substituted by one or more alkyl radicals, a naphthyl radical optionally substituted by one or more alkyl radicals, an indolyl radical optionally substituted by one or more alkyl radicals or a phenyl radical optionally substituted by one or more substituents chosen from halogen atoms and alkyl, alkoxy, hydroxyl, nitro, amino, monoalkylamino, dialkylamino, alkoxycarbonyl, -CO-NR₇R₈, -NH-CO-CH₃, trifluoromethyl or trifluoromethoxy radicals, and it being also understood that, except when otherwise mentioned, the alkyl, alkylene and alkoxy radicals and the alkyl, alkylene and alkoxy portions contain 1 to 4 carbon atoms in a straight or branched chain, the acyl radicals or portions contain 2 to 4 carbon atoms and the cycloalkyl radicals and portions contain 3 to 6 carbon atoms,
and their salts and their isomers when they contain at least one asymmetric centre.

2. Compounds of formula (I) according to claim 1 for which R₁ represents an isopropenyl, cyclohexyl, tetrahydrophenyl, cyclopentadienyl, dihydrophenyl, norbornyl, adamantyl or norbornenyl radical, and their salts and their isomers when they contain at least one asymmetric centre.

3. Compounds of formula (I) according to claim 1 for which R₇ and R₈ form, with the nitrogen atom to which they are attached, a heterocycle chosen from piperidino rings optionally substitued by one or more alkyl radicals or a 1,2,3,4-tetrahydroquinoline ring-system.

4. Compounds of formula (I) according to claim 1 for which R₉ and R₁₀ form, with the nitrogen atom to which they are attached, a heterocycle chosen from piperidino, perhydro-1-azepinyl, 1,2,3,6-tetrahydro-1-pyridyl, 1,2,3,4-tetrahydro-1-quinolyl, 1-pyrrolidinyl, 1,2,3,4-tetrahydro-2-isoquinolyl, thiomorpholino or 1-indolinyl ring-systems, it being possible for these rings to be optionally substituted by at least one alkyl radical.

5. Compounds of formula (I) according to claim 1 for which R represents a hydrogen atom or an alkyl or phenyl radical, R₁ represents an alkyl or phenyl radical, R₂ represents a -(CH₂)ₙ-CO-R₆ chain, R₃ represents a hydrogen atom or an alkyl radical, R₄ represents an alkyl radical, R₅ represents a phenylamino radical in which the phenyl ring is substituted by a carboxyl radical, R₆ represents an alkoxy radical and n is equal to 0, their salts and their isomers.

6. The following compounds of formula (I) according to claim 1:
- (S)-3-{3-[2-(2-tert-butoxycarbonyl-5,5-diphenyl-1-pyrrolidinyl)-2-oxoethyl]ureido}benzoic acid,
- (2S,5R)-3-{3-[2-(2-tert-butoxycarbonyl-5-methyl-5-phenyl-1-pyrrolidinyl)-2-oxoethyl]ureido}benzoic acid,
- (2RS,5SR)-3-{3-[2-(2-tert-butoxycarbonyl-2-methyl-5-phenyl-1-pyrrolidinyl)-2-oxoethyl]ureido}benzoic acid,
- (2S,5S)-3-{3-[2-(2-tert-butoxycarbonyl-5-butyl-1-pyrrolidinyl)-2-oxoethyl]ureido}benzoic acid,
their salts and their isomers.

7. Process for the preparation of the compounds of formula (I) according to claim 1 for which R₅ represents an optionally substituted phenylamino radical, characterized in that a reactive derivative of carbamic acid, optionally obtained in situ by reacting a reactive derivative of carbonic acid, chosen from N,N'-carbonyldiimidazole, phosgene, diphosgene, triphosgene and p-nitrophenyl chloroformate, with a derivative of formula: 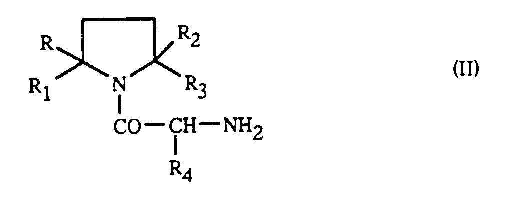 in which R, R₁, R₂, R₃ and R₄ have the same meanings as in claim 1, is reacted with an aniline in which the phenyl ring is optionally substituted by one or more substituents chosen from halogen atoms and alkyl, alkoxy, alkylthio, trifluoromethyl, carboxyl, alkoxycarbonyl, hydroxyl, nitro, amino, acyl, cyano, sulphamoyl, carbamoyl, hydroxyiminoalkyl, alkoxyiminoalkyl, hydroxyaminocarbonyl, alkoxyaminocarbonyl, 5-tetrazolyl, 5-tetrazolylalkyl, trifluoromethylsulphonamido, alkylsulphinyl, mono- or polyhydroxyalkyl, sulpho, -alk-O-CO-alk, -alk-COOX, -alk-O-alk, -alk'-COOX, -O-alk-COOX, -CH=CH-COOX, -CO-COOX, -alk-SO₃H in salt form, -CH=CH-alk', -C(=NOH)-COOX, -S-alk-COOX, -SO-alk-COOX, -SO₂-alk-COOX, -O-CH₂-alk'- COOX, -CX=N-O-alk-COOX, -alk-N(OH)-CO-alk, -alk-SO₂H, -SO₂-NH-CO-R₁₁, -SO₂-NH-SO₂-R₁₁, -CO-NH-CO-R₁₁, -CO-NH-SO₂-R₁₁, -B(OH)₂, -C(NH₂)=NOH, -SO₂-NH-R₁₂, -CO-NH-R₁₂, 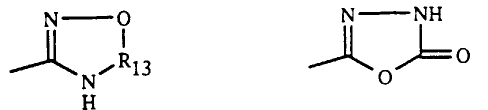 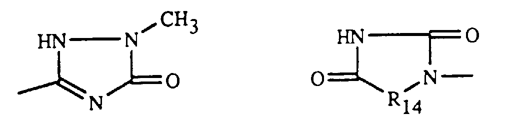 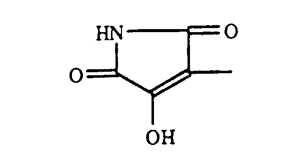 or 2,2-dimethyl-4,6-dioxo-1,3-dioxan-5-yl radicals, and the product is isolated and optionally converted to a salt.

8. Process for the preparation of the compounds of formula (I) according to claim 1 for which R₅ represents a phenylamino radical in which the phenyl ring is optionally substituted by one or more substituents chosen from halogen atoms and alkyl, alkoxy, alkylthio, trifluoromethyl, nitro, acyl, cyano, sulphamoyl, alkoxycarbonyl, carbamoyl, alkoxyiminoalkyl, alkoxyaminocarbonyl, -alk-O-CO-alk, -CH=CH-alk', -alk-O-alk, trifluoromethylsulphonamido, -alk-SO₃H (in salt form), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX or -alk'-COOX radicals in which X is other than a hydrogen atom, characterized in that a derivative of formula: 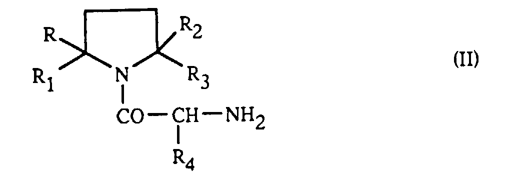 in which R, R₁, R₂, R₃ and R₄ have the same meanings as in claim 1, is reacted with a phenyl isocyanate in which the phenyl ring is optionally substituted by one or more substituents chosen from halogen atoms and alkyl, alkoxy, alkylthio, trifluoromethyl, nitro, acyl, cyano, sulphamoyl, alkoxycarbonyl, carbamoyl, alkoxyiminoalkyl, alkoxyaminocarbonyl, -alk-O-CO-alk, -CH=CH-alk', -alk-O-alk, trifluoromethylsulphonamido, -alk-SO₃H (in salt form), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX or -alk'-COOX radicals in which X is other than a hydrogen atom, and the product is isolated and optionally converted to a salt.

9. Process for the preparation of the compounds of formula (I) according to claim 1 for which R₅ represents a naphthyl, indolyl, quinolyl or optionally substituted phenyl radical, characterized in that a derivative of formula: 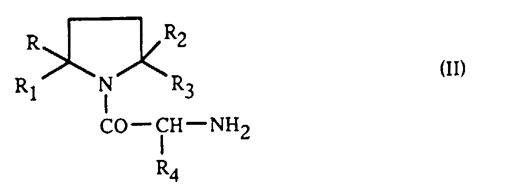 in which R, R₁, R₂, R₃ and R₄ have the same meanings as in claim 1, is reacted with an acid of formula HOOC-R₅ in which R₅ has the same meanings as above, or a reactive derivative of this acid, and the product is isolated and optionally converted to a salt.

10. Process for the preparation of the compounds of formula (I) according to claim 1, for which R₅ represents a phenylamino radical in which the phenyl ring is substituted by a carboxyl, -alk-COOH, -O-alk-COOH, -alk'-COOH, -CH=CH-COOH, -CO-COOH, -S-alk-COOH, -SO-alk-COOH, -SO₂-alk-COOH, -C(=NOH)-COOH, -O-CH₂-alk'-COOH or -CX=N-O-alk-COOH radical and/or R₂ represents a -(CH₂)ₙ-COOH chain, characterized in that a corresponding ester of formula (I) is hydrolysed or, according to the situation, subjected to hydrogenolysis, and the product is isolated and optionally converted to a salt.

11. Process for the preparation of the compounds of formula (I) according to claim 1 for which R₅ represents a phenylamino radical in which the phenyl ring is substituted by a hydroxyiminoalkyl or alkoxyiminoalkyl radical, characterized in that a corresponding acylated derivative of formula (I) is reacted with a derivative of formula:
H₂N-OR₁₈ (XIV)
in which R₁₈ represents a hydrogen atom or an alkyl radical, and the product is isolated and optionally converted to a salt.

12. Process for the preparation of the compounds of formula (I) according to claim 1 for which R₂ represents a -(CH₂)ₙ-CO-R₆ chain, R₆ not being a hydroxyl radical, and R₅ represents a naphthyl, indolyl, quinolyl or optionally substituted phenyl radical or a phenylamino radical in which the phenyl ring is optionally substituted by one or more substituents chosen from halogen atoms and alkyl, alkoxy, alkylthio, trifluoromethyl, nitro, acyl, cyano, sulphamoyl, alkoxycarbonyl, carbamoyl, alkoxyiminoalkyl, alkoxyaminocarbonyl, -alk-O-CO-alk, -CH=CH-alk', -alk-O-alk, trifluoromethylsulphonamido, -alk-SO₃H (in salt form), -O-alk-COOX, -CH=CH-COOX, -CO-COOX, -S-alk-COOX, -SO-alk-COOX, -SO₂-alk-COOX, -O-CH₂-alk'-COOX, -CX=N-O-alk-COOX, -alk-COOX or -alk'-COOX radicals in which X is other than a hydrogen atom, characterized in that a derivative of formula: 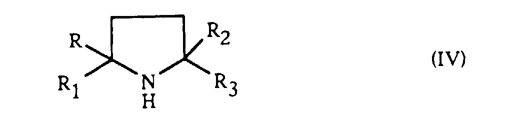 in which R, R₁ and R₃ have the same meanings as in claim 1 and R₂ has the same meanings as above, is reacted with an acid of formula: 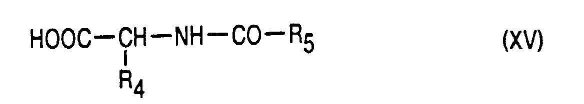 in which R₅ has the same meanings as above, or a reactive derivative of this acid, and R₄ has the same meanings as in claim 1, and the product is isolated and optionally converted to a salt.

13. Medicaments, characterized in that they contain, as active principle, at least one of the compounds according to one of claims 1 to 6.

14. Medicaments according to claim 13 which can be used for the treatment of disorders linked to CCK and to gastrin.
